# EUROPEAN PATENT APPLICATION

(11) **EP 3 336 545 A1**
(43) Date of publication of application: **20.06.2018**
(21) Application number: 16203689.1
(22) Date of filing: 13.12.2016
(51) Int. Cl.: G01N 33/563

(54) **REVERSIBLE LABELING OF ANTIGENS IN BIOLOGICAL SPECIMENS**

(71) Applicant: Miltenyi Biotec GmbH, 51429 Bergisch Gladbach (DE)
(72) Inventor: NÖLLE, Volker, 51515 Kürten (DE); PANKRATZ, Jennifer, 51103 Köln (DE); GÜNTHER, Gritt, 50670 Köln (DE)
(74) Representative: Biervert, Christian

(57) **Abstract**

The present invention provides a method for reversible labeling an antigen A of a biological specimen comprising said antigen A with a monovalent antigen binding molecule K comprising a binding site B for specifically binding said antigen A, the method comprising the steps a) contacting said biological specimen comprising said antigen A with said antigen binding molecule K, thereby generating a complex of A/K, b) removing the monovalent antigen binding molecule K that is not bound in said complex of A/K, wherein the equilibrium dissociation constant (K_{D}) for the binding of said antigen A and said monovalent antigen binding molecule K has a value of equal or greater than 0.5E-08 M and wherein the dissociation rate constant (k(off)) of said binding has a value of equal or greater than 1E-03 sec-1. In some embodiments of the invention a multimerization component F for multimerization of monovalent antigen binding molecules (F/K complex) is included in the method, wherein said F/K complex can be disrupted.

SEQUENCE LISTING

<110>  Miltenyi Biotec GmbH

<120>  Reversible labeling of antigens in biological specimens

<130>  Mil_103

<160>  22

<170>  PatentIn version 3.5

<210>  1
<211>  119
<212>  PRT
<213>  Artificial sequence

<220>
<223>  heavy chain variable domain of OKT3-hum

<400>  1

Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
                85                  90                  95

Ala Arg Tyr Tyr Asp Asp His Tyr Ser Leu Asp Tyr Trp Gly Gln Gly
            100                 105                 110

Thr Pro Val Thr Val Ser Ser
        115

<210>  2
<211>  106
<212>  PRT
<213>  Artificial sequence

<220>
<223>  light chain variable domain of OKT3-hum

<400>  2

<210>  3
<211>  117
<212>  PRT
<213>  Artificial sequence

<220>
<223>  heavy chain variable domain of 13B8.2

<400>  3

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Thr Phe
            20                  25                  30

Gly Val His Trp Val Arg Gln Ser Pro Gly Lys Gly Leu Glu Trp Leu
        35                  40                  45

Gly Val Ile Trp Arg Ser Gly Ile Thr Asp Tyr Asn Val Pro Phe Met
    50                  55                  60

Ser Arg Leu Ser Ile Thr Lys Asp Asn Ser Lys Ser Gln Val Phe Phe
65                  70                  75                  80

Lys Leu Asn Ser Leu Gln Pro Asp Asp Thr Ala Ile Tyr Tyr Cys Ala
                85                  90                  95

Lys Asn Asp Pro Gly Thr Gly Phe Ala Tyr Trp Gly Gln Gly Thr Leu
            100                 105                 110

Val Thr Val Ser Ala
        115

<210>  4
<211>  107
<212>  PRT
<213>  Artificial sequence

<220>
<223>  light chain variable domain of 13B8.2

<400>  4

Asp Ile Gln Met Thr Gln Ser Pro Ala Ser Leu Ser Ala Ser Val Gly
1               5                   10                  15

Glu Thr Val Thr Phe Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

His Asp Ala Lys Thr Leu Ala Glu Gly Val Pro Ser Arg Phe Ser Gly
    50                  55                  60

Gly Gly Ser Gly Thr Gln Phe Ser Leu Lys Ile Asn Thr Leu Gln Pro
65                  70                  75                  80

Glu Asp Phe Gly Thr Tyr Tyr Cys Gln His His Tyr Gly Asn Pro Pro
                85                  90                  95

Thr Phe Gly Gly Gly Thr Lys Leu Glu Ile Lys
            100                 105

<210>  5
<211>  117
<212>  PRT
<213>  Artificial sequence

<220>
<223>  heavy chain variable domain of 13B8.2-Y92A

<400>  5

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Thr Phe
            20                  25                  30

Gly Val His Trp Val Arg Gln Ser Pro Gly Lys Gly Leu Glu Trp Leu
        35                  40                  45

Gly Val Ile Trp Arg Ser Gly Ile Thr Asp Tyr Asn Val Pro Phe Met
    50                  55                  60

Ser Arg Leu Ser Ile Thr Lys Asp Asn Ser Lys Ser Gln Val Phe Phe
65                  70                  75                  80

Lys Leu Asn Ser Leu Gln Pro Asp Asp Thr Ala Ile Tyr Tyr Cys Ala
                85                  90                  95

Lys Asn Asp Pro Gly Thr Gly Phe Ala Tyr Trp Gly Gln Gly Thr Leu
            100                 105                 110

Val Thr Val Ser Ala
        115

<210> 6
<211> 107
<212> PRT
<213> Artificial sequence

<220>
<223> light chain variable domain of 13B8.2-Y92A

<400> 6

Asp Ile Gln Met Thr Gln Ser Pro Ala Ser Leu Ser Ala Ser Val Gly
1               5                   10                  15

Glu Thr Val Thr Phe Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

His Asp Ala Lys Thr Leu Ala Glu Gly Val Pro Ser Arg Phe Ser Gly
    50                  55                  60

Gly Gly Ser Gly Thr Gln Phe Ser Leu Lys Ile Asn Thr Leu Gln Pro
65                  70                  75                  80

Glu Asp Phe Gly Thr Tyr Tyr Cys Gln His His Ala Gly Asn Pro Pro
                85                  90                  95

Thr Phe Gly Gly Gly Thr Lys Leu Glu Ile Lys
            100                 105

<210>  7
<211>  117
<212>  PRT
<213>  Artificial sequence

<220>
<223>  heavy chain variable domain of 13B8.2-F100A

<400>  7

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Thr Phe
            20                  25                  30

Gly Val His Trp Val Arg Gln Ser Pro Gly Lys Gly Leu Glu Trp Leu
        35                  40                  45

Gly Val Ile Trp Arg Ser Gly Ile Thr Asp Tyr Asn Val Pro Phe Met
    50                  55                  60

Ser Arg Leu Ser Ile Thr Lys Asp Asn Ser Lys Ser Gln Val Phe Phe
65                  70                  75                  80

Lys Leu Asn Ser Leu Gln Pro Asp Asp Thr Ala Ile Tyr Tyr Cys Ala
                85                  90                  95

Lys Asn Asp Pro Gly Thr Gly Ala Ala Tyr Trp Gly Gln Gly Thr Leu
            100                 105                 110

Val Thr Val Ser Ala
        115


<210>  8
<211>  107
<212>  PRT
<213>  Artificial sequence

<220>
<223>  light chain variable domain of 13B8.2-F100A

<400>  8

Asp Ile Gln Met Thr Gln Ser Pro Ala Ser Leu Ser Ala Ser Val Gly
1               5                   10                  15

Glu Thr Val Thr Phe Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

His Asp Ala Lys Thr Leu Ala Glu Gly Val Pro Ser Arg Phe Ser Gly
    50                  55                  60

Gly Gly Ser Gly Thr Gln Phe Ser Leu Lys Ile Asn Thr Leu Gln Pro
65                  70                  75                  80

Glu Asp Phe Gly Thr Tyr Tyr Cys Gln His His Tyr Gly Asn Pro Pro
                85                  90                  95

Thr Phe Gly Gly Gly Thr Lys Leu Glu Ile Lys
            100                 105

<210>  9
<211>  117
<212>  PRT
<213>  Artificial sequence

<220>
<223>  heavy chain variable domain of 13B8.2-LAV

<400>  9

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Thr Phe
            20                  25                  30

Gly Val His Trp Val Arg Gln Ser Pro Gly Lys Gly Leu Glu Trp Leu
        35                  40                  45

Gly Val Ile Trp Ala Ser Gly Ile Thr Asp Tyr Asn Val Pro Phe Met
    50                  55                  60

Ser Arg Leu Ser Ile Thr Lys Asp Asn Ser Lys Ser Gln Val Phe Phe
65                  70                  75                  80

Lys Leu Asn Ser Leu Gln Pro Asp Asp Thr Ala Ile Tyr Tyr Cys Ala
                85                  90                  95

Lys Asn Asp Pro Gly Thr Gly Phe Ala Tyr Trp Gly Gln Gly Thr Leu
            100                 105                 110

Val Thr Val Ser Ala
        115

<210>  10
<211>  107
<212>  PRT
<213>  Artificial sequence

<220>
<223>  light chain variable domain of 13B8.2-LAV

<400>  10

Asp Ile Gln Met Thr Gln Ser Pro Ala Ser Leu Ser Ala Ser Val Gly
1               5                   10                  15

Glu Thr Val Thr Phe Thr Cys Arg Ala Ser Glu Asn Ile Tyr Ser Tyr
            20                  25                  30

Leu Ala Trp Tyr Gln Gln Lys Gln Gly Lys Ser Pro Gln Leu Leu Val
        35                  40                  45

His Asp Ala Lys Thr Leu Ala Glu Gly Val Pro Ser Arg Phe Ser Gly
    50                  55                  60

Gly Gly Ser Gly Thr Gln Phe Ser Leu Lys Ile Asn Thr Leu Gln Pro
65                  70                  75                  80

Glu Asp Phe Gly Thr Tyr Tyr Cys Gln His Ala Tyr Gly Asn Pro Pro
                85                  90                  95

Thr Phe Gly Gly Gly Thr Lys Leu Glu Ile Lys
            100                 105


<210>  11
<211>  118
<212>  PRT
<213>  Artificial sequence

<220>
<223>  heavy chain variable domain of OKT8-LAV

<400>  11

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Thr
            20                  25                  30

Tyr Ile His Phe Val Arg Gln Arg Pro Glu Gln Gly Leu Glu Trp Ile
        35                  40                  45

Gly Arg Ile Asp Pro Ala Asn Asp Asn Thr Ile Tyr Ala Ser Lys Phe
    50                  55                  60

Gln Gly Lys Ala Thr Ile Thr Ala Asp Thr Ser Ser Asn Thr Ala Tyr
65                  70                  75                  80

Met His Leu Ser Ser Leu Thr Ser Gly Asp Thr Ala Val Tyr Tyr Cys
                85                  90                  95

Gly Arg Gly Tyr Gly Tyr Tyr Val Phe Asp His Trp Gly Gln Gly Thr
            100                 105                 110

Thr Val Thr Val Ser Ser
        115

<210>  12
<211>  107
<212>  PRT
<213>  Artificial sequence

<220>
<223>  light chain variable domain of OKT8-LAV

<400>  12

Asp Val Gln Ile Asn Gln Ser Pro Ser Phe Leu Ala Ala Ser Pro Gly
1               5                   10                  15

Glu Thr Ile Thr Ile Asn Cys Arg Thr Ser Arg Ser Ile Ser Gln Tyr
            20                  25                  30

Leu Ala Trp Tyr Gln Glu Lys Pro Gly Lys Thr Asn Lys Leu Leu Ile
        35                  40                  45

Tyr Ser Gly Ser Thr Leu Gln Ser Gly Ile Pro Ser Arg Phe Ser Gly
    50                  55                  60

Ser Gly Ser Gly Thr Asp Phe Thr Leu Thr Ile Ser Gly Leu Glu Pro
65                  70                  75                  80

Glu Asp Phe Ala Met Tyr Tyr Cys Gln Gln Ala Asn Glu Asn Pro Leu
                85                  90                  95

Thr Phe Gly Ala Gly Thr Lys Leu Glu Leu Arg
            100                 105

<210>  13
<211>  117
<212>  PRT
<213>  Artificial sequence

<220>
<223>  heavy chain variable domain of RFT5

<400>  13

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Ser Phe Thr Arg Tyr
            20                  25                  30

Trp Met His Trp Ile Lys Gln Arg Pro Gly Gln Gly Leu Glu Trp Ile
        35                  40                  45

Gly Ala Ile Tyr Pro Gly Asn Ser Asp Thr Ser Tyr Asn Gln Lys Phe
    50                  55                  60

Glu Gly Lys Ala Lys Leu Thr Ala Val Thr Ser Ala Ser Thr Ala Tyr
65                  70                  75                  80

Met Glu Leu Ser Ser Leu Thr His Glu Asp Ser Ala Val Tyr Tyr Cys
                85                  90                  95

Ser Arg Asp Tyr Gly Tyr Tyr Phe Asp Phe Trp Gly Gln Gly Thr Thr
            100                 105                 110

Leu Thr Val Ser Ser
        115

<210> 14
<211> 104
<212> PRT
<213> Artificial sequence

<220>
<223> light chain variable domain of RFT5

<400> 14

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Ile Ser Tyr Met
            20                  25                  30

Gln Trp Tyr Gln Gln Lys Pro Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys His Gln Arg Ser Ser Tyr Thr Phe Gly
                85                  90                  95

Gly Gly Thr Lys Leu Glu Ile Lys
            100

<210>  15
<211>  117
<212>  PRT
<213>  Artificial sequence

<220>
<223>  heavy chain variable domain of RFT5-LAV

<400>  15

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Ser Phe Thr Arg Tyr
            20                  25                  30

Trp Met Ala Trp Ile Lys Gln Arg Pro Gly Gln Gly Leu Glu Trp Ile
        35                  40                  45

Gly Ala Ile Tyr Pro Gly Asn Ser Asp Thr Ser Tyr Asn Gln Lys Phe
    50                  55                  60

Glu Gly Lys Ala Lys Leu Thr Ala Val Thr Ser Ala Ser Thr Ala Tyr
65                  70                  75                  80

Met Glu Leu Ser Ser Leu Thr His Glu Asp Ser Ala Val Tyr Tyr Cys
                85                  90                  95

Ser Arg Asp Tyr Gly Tyr Tyr Phe Asp Phe Ala Gly Gln Gly Thr Thr
            100                 105                 110

Leu Thr Val Ser Ser
        115

<210>  16
<211>  104
<212>  PRT
<213>  Artificial sequence

<220>
<223>  light chain variable domain of RFT5-LAV

<400>  16

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Ile Ser Tyr Met
            20                  25                  30

Gln Trp Tyr Gln Gln Lys Pro Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys His Gln Arg Ser Ser Tyr Thr Phe Gly
                85                  90                  95

Gly Gly Thr Lys Leu Glu Ile Lys
            100

<210>  17
<211>  123
<212>  PRT
<213>  Artificial sequence

<220>
<223>  heavy chain variable domain of 15E8-hum

<400>  17

Gln Val Gln Leu Gln Glu Ser Gly Pro Gly Leu Val Lys Pro Ser Glu
1               5                   10                  15

Thr Leu Ser Leu Thr Cys Thr Val Ser Gly Phe Ser Leu Thr Ser Tyr
            20                  25                  30

Gly Val His Trp Ile Arg Gln Pro Pro Gly Lys Gly Leu Glu Trp Leu
        35                  40                  45

Gly Val Ile Trp Ala Gly Gly Ser Thr Asn Tyr Asn Ser Ser Leu Lys
    50                  55                  60

Ser Arg Val Thr Ile Ser Lys Asp Thr Ser Lys Asn Gln Val Ser Leu
65                  70                  75                  80

Lys Leu Ser Ser Val Thr Ala Ala Asp Thr Ala Val Tyr Tyr Cys Ala
                85                  90                  95

Arg Asp Lys Arg Ala Pro Gly Lys Leu Tyr Tyr Gly Tyr Pro Asp Tyr
            100                 105                 110

Trp Gly Gln Gly Thr Thr Leu Thr Val Ser Ser
        115                 120

<210>  18
<211>  111
<212>  PRT
<213>  Artificial sequence

<220>
<223>  light chain variable domain of 15E8-hum

<400>  18

Asp Ile Val Met Thr Gln Ser Pro Asp Ser Leu Ala Val Ser Leu Gly
1               5                   10                  15

Glu Arg Ala Thr Ile Asn Cys Arg Ala Ser Glu Ser Val Glu Tyr Tyr
            20                  25                  30

Val Thr Ser Leu Met Gln Trp Tyr Gln Gln Lys Pro Gly Gln Pro Pro
        35                  40                  45

Lys Leu Leu Ile Tyr Ala Ala Ser Asn Val Glu Ser Gly Val Pro Ala
    50                  55                  60

Arg Phe Ser Gly Ser Gly Ser Gly Thr Asp Phe Thr Leu Thr Ile Ser
65                  70                  75                  80

Pro Leu Gln Ala Glu Asp Val Ala Val Tyr Tyr Cys Gln Gln Thr Arg
                85                  90                  95

Lys Val Pro Ser Thr Phe Gly Gly Gly Thr Lys Leu Glu Ile Lys
            100                 105                 110


<210>  19
<211>  117
<212>  PRT
<213>  Artificial sequence

<220>
<223>  heavy chain variable domain of MEM56-LAV

<400>  19

Gln Val Gln Leu Gln Gln Ser Gly Ala Glu Leu Ala Lys Pro Gly Ala
1               5                   10                  15

Ser Val Lys Met Ser Cys Lys Ala Ser Gly Tyr Asn Phe Thr Arg Tyr
            20                  25                  30

Trp Met His Trp Ile Lys Gln Arg Pro Gly Gln Gly Leu Glu Trp Ile
        35                  40                  45

Gly Tyr Ile Asn Pro Asn Thr Gly His Thr Glu Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Thr Tyr Tyr Tyr Gly Ser Arg Arg Asp Ala Gly Gln Gly Thr Thr
            100                 105                 110

Leu Thr Val Ser Ser
        115

<210>  20
<211>  108
<212>  PRT
<213>  Artificial sequence

<220>
<223>  light chain variable domain of MEM56-LAV

<400>  20

Gln Ile Val Leu Thr Gln Ser Pro Ala Ile Met Ser Ala Ser Leu Gly
1               5                   10                  15

Glu Arg Val Thr Met Thr Cys Thr Thr Ser Ser Ser Val Ser Ser Ser
            20                  25                  30

Tyr Leu His Trp Tyr Gln Gln Lys Pro Gly Ser Ser Pro Lys Leu Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Ser Tyr Ser Leu Thr Ile Ser Thr Val Glu
65                  70                  75                  80

Ala Glu Asp Ala Ala Thr Tyr Tyr Cys His Gln Tyr His Arg Ser Pro
                85                  90                  95

Leu Thr Phe Gly Ala Gly Thr Lys Leu Glu Leu Lys
            100                 105

<210>  21
<211>  103
<212>  PRT
<213>  Artificial sequence

<220>
<223>  human IgG1 heavy chain constant region CH1

<400>  21

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys
            100

<210>  22
<211>  107
<212>  PRT
<213>  Artificial sequence

<220>
<223>  human kappa light chain constant region

<400>  22

## Description

### Field of invention

The present invention generally relates to the field of labeling of an antigen in a biological specimen with a monovalent antigen binding molecule, in particular to reversible labeling of an antigen in a biological specimen.

### Background of the invention

Labeling of antigens is used in several applications such as the identification and characterization of antigens and cell populations comprising antigens, detection of subcellular structures and extracellular matrix components, cell sorting, detection and monitoring of cancer antigens, immunolabeling, imaging, and immunocytochemistry. The removal of the label is of interest in applications where the label interferes with subsequent process steps. For example, during separation and isolation of cells it may be useful to remove the label from the cell-bound antigen to either allow multiple labeling and cell sorting steps or to generate "label-free" cells for cell therapeutic applications. In cell histology, a removal of the label from the cell- or matrix-bound antigen would allow the serial staining of hundreds of different antigens on a single sample without the need to use hundreds of different detection moieties.

US7776562 discloses a method for the reversible staining of a target cell with a detectable label, which makes use of monovalent conjugates, each monovalent conjugate comprising a single ligand which specifically binds a receptor molecule on the target cells, wherein the equilibrium dissociation constant K_{D} for the binding between said ligand of said monovalent conjugate and said receptor molecule is in the range of 1E-02 and 1E-07 M, and wherein the ligand and the detectable label are reversibly bound to each other using streptavidin / avidin, biotin, and analogs thereof. A disadvantage of said method is that it is not essential for the reversibility of such a method that a low affinity binding is given between a receptor binding reagent and a receptor molecule on the surface of the target cell, as also described in US9023604 by the same inventors.

US9023604 discloses a method of reversibly staining a target cell with a detectable label, which makes use of a receptor binding reagent, the receptor binding reagent comprising at least one binding site, wherein the binding site specifically binds to a receptor molecule, wherein the dissociation rate constant k(off) for the binding between said receptor binding reagent via the binding site and said receptor molecule has a value of about 0.5E-04 sec-1 or greater, and wherein the receptor binding reagent and the detectable label are reversibly bound to each other using a multimerization reagent. The method disclosed in US9023604 has the disadvantage that not all receptor binding reagents that have a k(off) value of about 0.5E-04 sec-1 or greater are sufficiently reversible in binding to the receptor molecule to carry out the therein disclosed method.

US7482000 discloses mutant Fab fragments of the anti-CD4 antibody 13B8.2 and their kinetic parameters of CD4 binding (k(on), k(off), K_{D}) using immobilized CD4 antigen and surface plasmon resonance.

US5965456 discloses an apparatus for detecting the presence of an analyte of interest (i.e. an isolated antigen or ligand) in a solution sample, comprising an immobilized binding partner comprising a solid support and a reversibly binding receptor which is capable of binding to the analyte of interest, wherein the receptor/analyte complex has a dissociation rate constant k(off) of greater than 3.2E-2 per second such that the apparatus responds rapidly to changes in analyte concentration and is suitable for on-line, real time measurement of the analyte.

There is need in the art for an improved or alternative method of reversibly labeling of antigens in biological specimens.

### Summary of the invention

We identified Fab molecules that enable reversible labeling having K_{D} values outside the range of 1E-02 and 1E-07 M, although this range is disclosed in US7776562 to be sufficient for the reversibility of a binding between an antigen binding molecule and its antigen (see examples 6, 8, 9, and 10: 8 out of 15 Fab molecules having K_{D} values < 1E-07 M enable reversible labeling). In addition, we identified Fab molecules having k(off) values of about 0.5E-04 sec-1 or greater that do not enable reversible labeling although this range is disclosed in US9023604 to be sufficient for the reversibility of a binding between an antigen binding molecule and its antigen (see examples 6, 8, 9, and 10: 8 out of 20 Fab molecules having k(off) values of 0.5E-04 sec-1 or greater do not enable reversible labeling).

We surprisingly found that monovalent antigen binding molecules having an equilibrium dissociation constant (K_{D}) within a well-defined range and having in addition a dissociation rate constant (k(off)) within a well-defined range can be used for reversible labeling of antigens recognized by said monovalent antigen binding molecules. From 12 Fabs as used herein that fulfil the criteria disclosed herein, 8 of said 12 Fabs did not fulfil the criteria of US7776562. From 8 Fabs as used herein that do not fulfil the criteria disclosed herein, all of said 8 Fabs fulfil the criteria of US9023604.

The range for the equilibrium dissociation constant (K_{D}) is equal or greater than 0.5E-08 M and the range for dissociation rate constant (k(off)) is equal or greater than 1E-03 sec-1, preferentially, the range for the equilibrium dissociation constant (K_{D}) is between 0.5E-08 M and 1E-04 M and the range for dissociation rate constant (k(off)) is between 1E-03 sec-1 and 1E-00 sec-1.

The combination of said ranges is essential for a monovalent antigen binding molecule so that it can be used for reversible labeling of antigens as disclosed herein. It is not sufficient that a monovalent antigen binding molecule fulfills only one of both ranges (i.e. either K_{D} or k(off)). Monovalent antigen binding molecules having K_{D} and k(off) values within both said ranges can be used reliably in methods for reversible labeling antigens in biological specimen, both in simple labeling procedures and in more complex procedures comprising multimerization components that can be disrupted. Monovalent antigen binding molecules having K_{D} and k(off) values within both said ranges allow for influencing the chemical equilibrium of free monovalent antigen binding molecules and monovalent antigen binding molecules bound to the antigens in a biological specimen when the free monovalent antigen binding molecules are removed, for example by using a washing step. This effect can be exploited for methods of reversibly labeling antigens in biological specimen by removing at least once the non-bound monovalent antigen binding molecules in the biological specimen.

### Brief description of the drawings

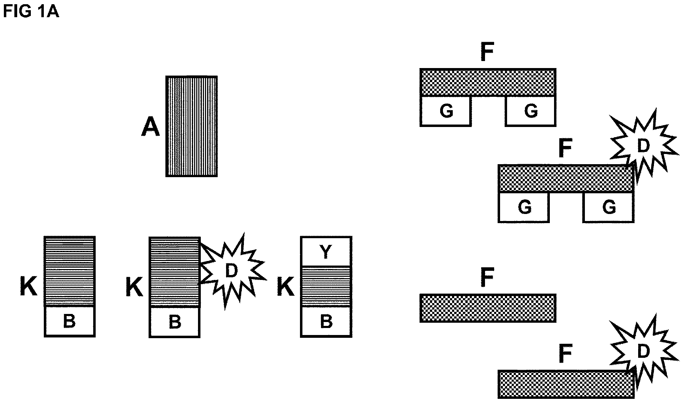

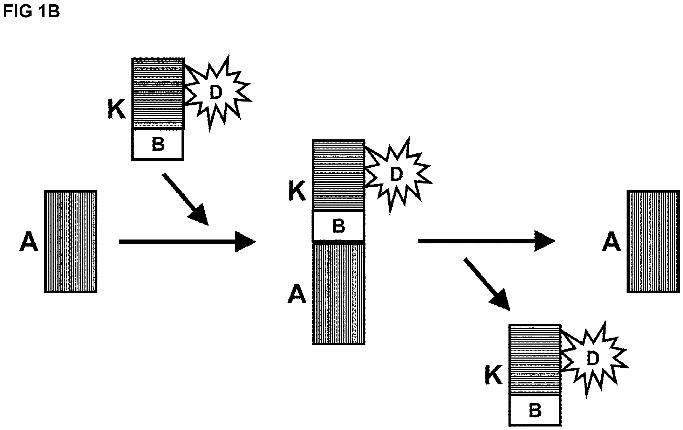

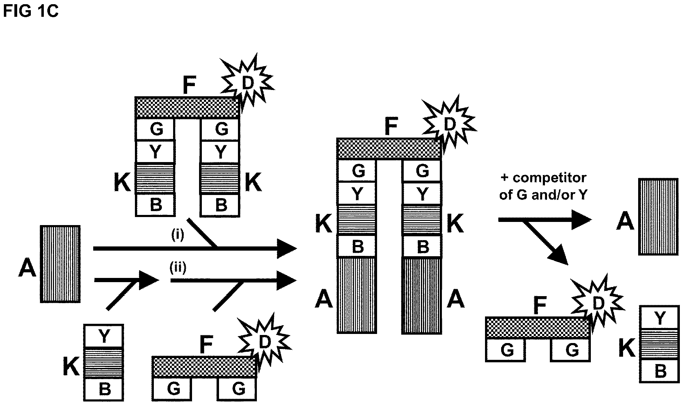

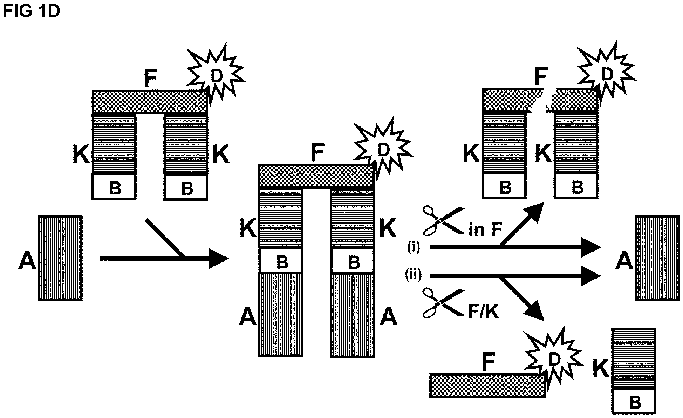

FIG 1: Principle of reversible labeling of antigens in biological specimen.
   A: Description of molecules. A, antigen (in a biological specimen); K, monovalent antigen binding molecule comprising a binding site B for specific binding of the antigen, and in some examples also comprising at least one binding site Y for non-covalent coupling to a multimerization component F; F, multimerization component, in some examples comprising at least two binding sites G for non-covalent coupling of F to K via the binding sites G and Y; D, detection moiety, may be coupled to either K or F. In all variants, coupling of D to K or F may be direct or indirect (for example by using dextran as a multimerization component and an anti-dextran antibody coupled to a fluorophore), and coupling of D to K or F may be covalent or non-covalent. In some examples, D may be coupled to K or F after contacting the antigen with K or a with complex comprising K and F. The following figures comprise exemplary variants.
   B: Reversible labeling of an antigen A using a monovalent antigen binding molecule K comprising binding site B for specifically binding said antigen A, forming the complex A/K. K may be coupled to a detection moiety D for analyzing and/or isolating. K is then removed from A.
   C: Reversible labeling of an antigen A using a monovalent antigen binding molecule K comprising binding a site B for specifically binding said antigen A and at least one binding site Y for coupling to a multimerization component F. Said F comprises at least two binding sites G for coupling to at least two molecules of said K in order to increase avidity of binding K to A. F and K are coupled non-covalently via the binding sites G and Y. F may be covalently or non-covalently coupled to a detection moiety D. The contacting is conducted either (i) by subsequently contacting F and K to form the complex K/F, and then contacting A with K/F to form the complex A/K/F, or (ii) by subsequently contacting A and K to form a complex A/K, and then contacting A/K with F to form a complex A/K/F. The complex K/F is disrupted to monomerize K, and K is then removed from A. Said disruption may be conducted by weakening the interaction between F and K (for example by adding a competitor competing with G and/or Y), and/or by fragmenting F in order to monomerize K (for example by enzymatic digestion of F, not shown), and/or by changing the conformation of F by a stimulus in order to monomerize K (for example by addition or removal of Ca²⁺ to induce a conformational change in a Ca²⁺-responsive multimerization component F, not shown).
   D: Reversible labeling of an antigen A using a monovalent antigen binding molecule K comprising a binding site B for specifically binding said antigen A. At least two molecules of said K are coupled covalently to a multimerization component F in order to increase avidity of binding K to A. F may be covalently or non-covalently coupled to a detection moiety D. A is contacted with K/F to form the complex A/K/F. Said complex K/F is disrupted to monomerize K, and K is then removed from A. Said disruption may be conducted (i) by fragmenting F in order to monomerize K (for example by enzymatic digestion of F), and/or (ii) by cleaving the covalent binding between F and K (for example by enzymatic, chemical, or photochemical cleavage, or mechanical shearing).

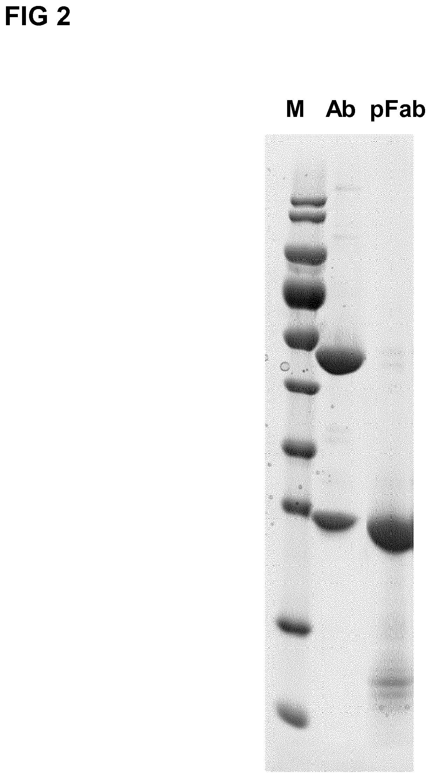

FIG 2: Coomassie-stained SDS gel (reducing conditions) with exemplary samples of the generation of a Fab fragment by papain digestion of an antibody (anti-CD19 clone SJ25-C1). M, molecular weight marker with sizes of 170, 130, 100, 70, 55, 40, 35, 25, 15, and 10 kDa from top to bottom. Ab, antibody before papain digestion; pFab, Fab fragment after papain digestion and removal of the Fc part.

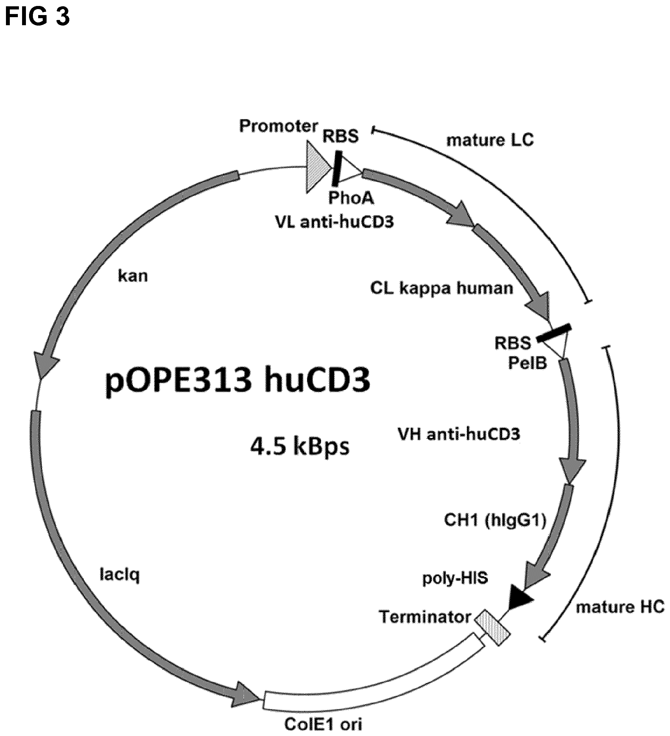

FIG 3: Plasmid map of the prokaryotic vector for the expression of Fab fragments in *E.coli*, exemplarily shown by an expression vector for an anti-CD3 rFab (rFab = recombinant Fab). LC, light chain; HC heavy chain; VL, light chain variable domain; VH, heavy chain variable domain; CL light chain constant domain; CH1 (hIgG1), CH1 domain of human IgG1; RBS, ribosome binding site; poly-HIS, poly-histidine sequence; kan, kanamycin resistance gene; lacIq, lac repressor gene; ori, origin of replication; PhoA, signal peptide PhoA; PelB, signal peptide PelB; kBps, kilo base pairs.

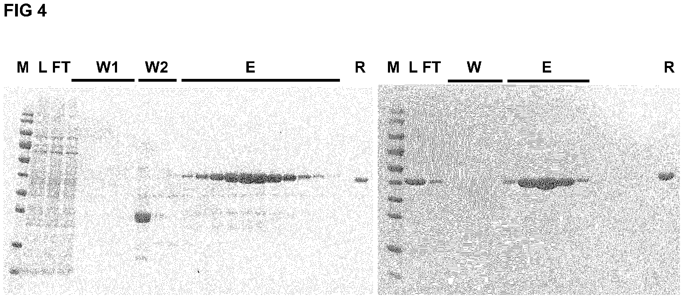

FIG 4: Coomassie-stained SDS gel (non-reducing conditions) with samples of different steps of the purification of recombinant anti-CD25 rFab (based on clone RFT5) as an example by metal chelate affinity (left side), followed by ion exchange chromatography (right side). M, molecular weight marker with sizes of 170, 130, 100, 70, 55, 40, 35, 25, 15, and 10 kDa from top to bottom. L, load (bacterial extract or eluate of metal chelate chromatography); FT, flow-through fraction; W, W1, W2, wash fractions; E, eluate factions; R, reference Fab (2 µg).

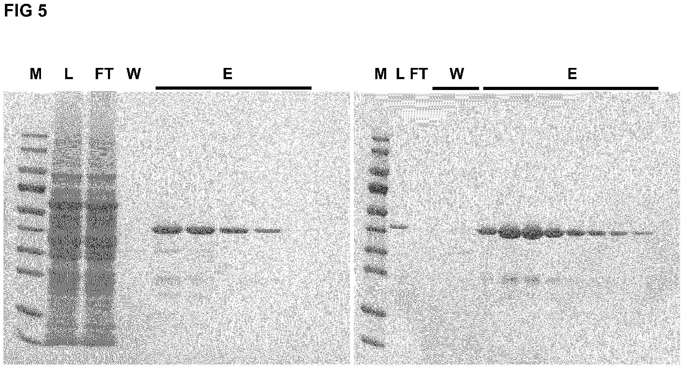

FIG 5: Coomassie-stained SDS gel (non-reducing conditions) with samples of different steps of the purification of a mutated variant of recombinant anti-CD25 rFab (RFT5-LAV, based on clone RFT5) as an example, by metal chelate affinity (left side), followed by ion exchange chromatography (right side). M, molecular weight marker with sizes of 170, 130, 100, 70, 55, 40, 35, 25, 15, and 10 kDa from top to bottom. L, load (bacterial extract or eluate of metal chelate chromatography); FT, flow-through fraction; W, wash fraction(s); E, eluate factions.

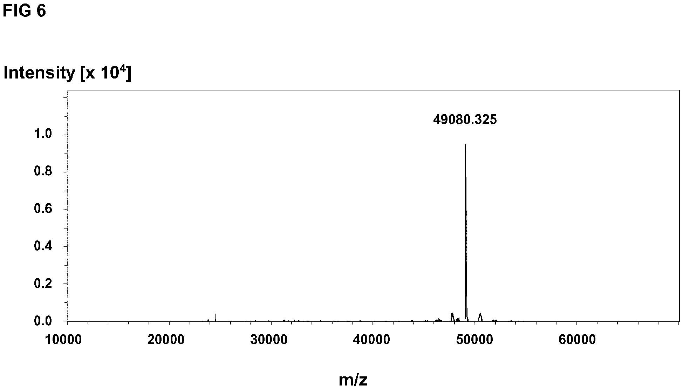

FIG 6: Intact mass determination of recombinant anti-CD25 Fab variant RFT5 rFab as an example by LC-MS (liquid chromatography-mass spectrometry). M/z, mass-to-charge-ratio.

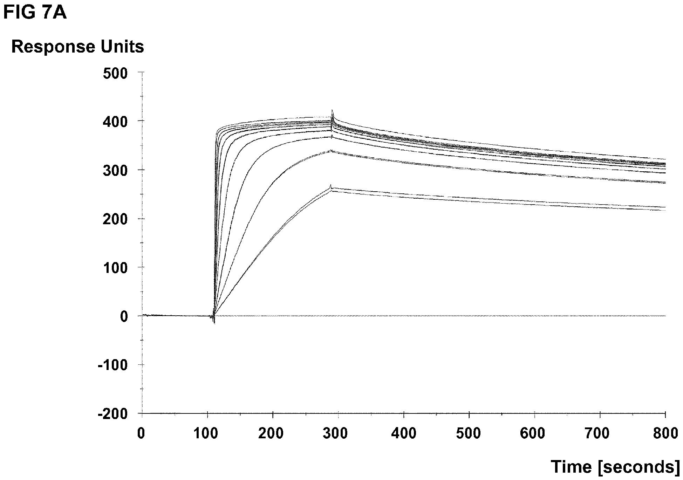

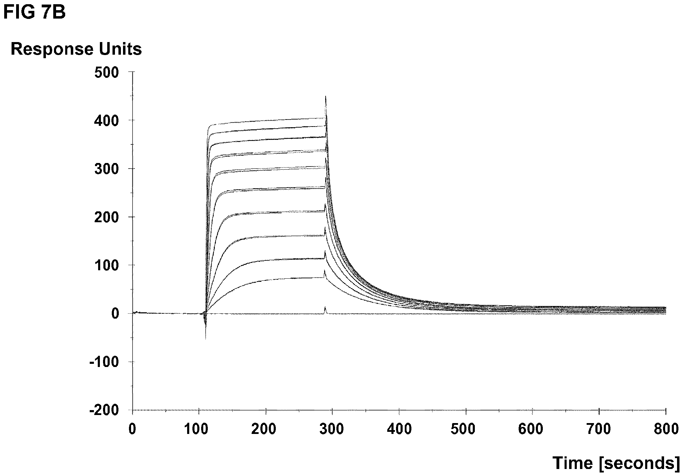

FIG 7: Surface plasmon resonance sensorgrams of anti-CD25 rFabs using immobilized recombinant CD25 antigen. At t ≈ 110 sec, different concentrations of Fab were injected for 180 sec. At t ≈ 290 sec, the dissociation phase started. A: RFT5 rFab as an example for an antigen binding molecule that does not enable reversible labeling. B: RFT5-LAV rFab as an example for an antigen binding molecule that enables reversible labeling.

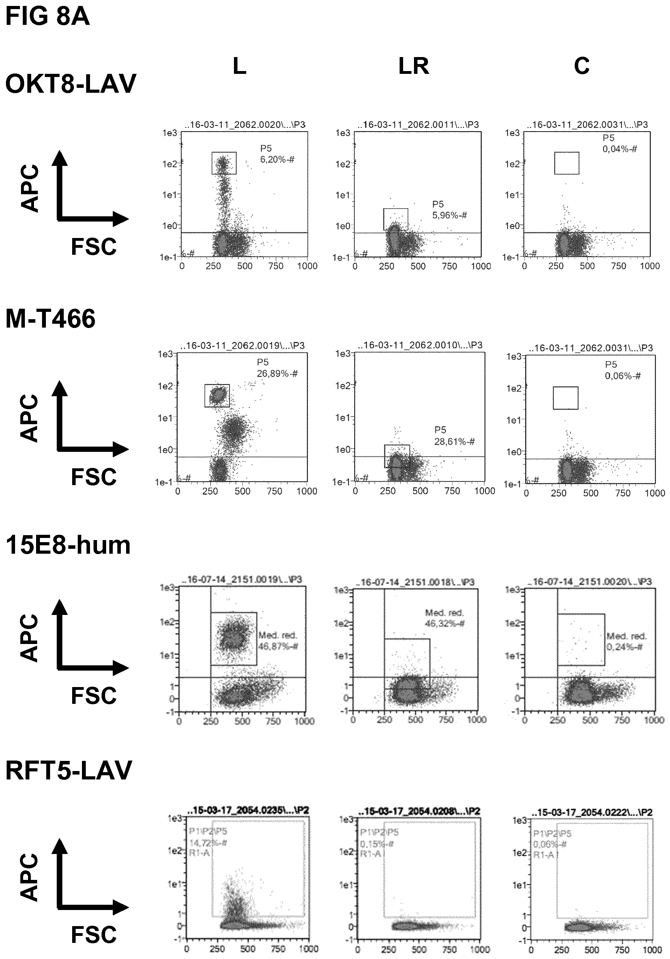

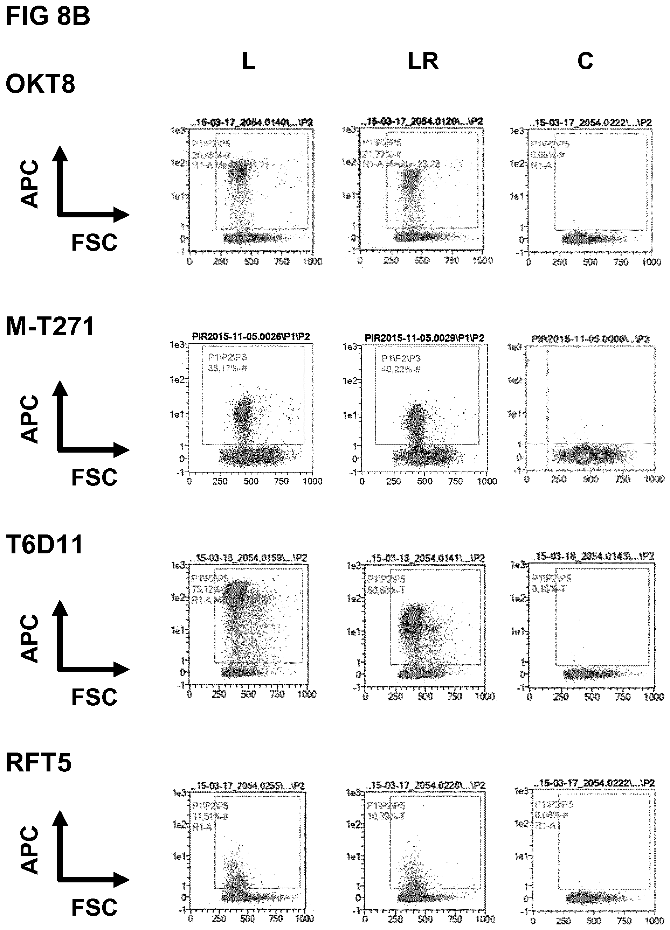

FIG 8: Flow cytometric analysis of reversibly labeled antigens in human PBMCs (peripheral blood mononuclear cells) using biotinylated Fabs as monovalent antigen binding molecules in combination with SA-APC (streptavidin-allophycocyanin) as detection moiety (added after Fab binding to the antigen, or after removal of the Fab from the antigen). A: Dot plots of Fabs that enable reversible labeling; B: Dot plots of Fabs that do not enable reversible labeling. Fab variants M-T466, OKT8, OKT8-LAV, RFT5, RFT5-LAV, M-T271, 15E8-hum, and T6D11 are shown as examples. Column L: labeled antigen (before removal); column LR: antigen after washing to remove Fab; column C: control staining with SA-APC, but without Fab. FSC: forward scatter. The range of the x-axis is from 0 to 1000, and of the y-axis from 1E-01 to 1E+03, or from -1 to 1E+03, respectively.

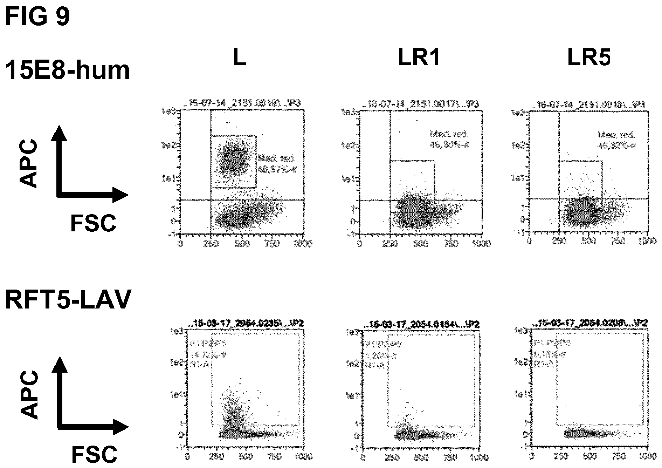

FIG 9: Flow cytometric analysis of reversibly labeled antigens in human PBMCs (peripheral blood mononuclear cells) using biotinylated Fabs as monovalent antigen binding molecules in combination with SA-APC (streptavidin-allophycocyanin) as detection moiety (added after Fab binding to the antigen, or after removal of the Fab from the antigen) and different numbers of washing steps. Dot plots of Fab variants 15E8-hum and RFT5-LAV are shown as examples. Column L: labeled antigen (before removal); column LR1: antigen after one washing step to remove Fab ; column LR5: antigen after five washing steps to remove Fab. FSC: forward scatter. The range of the x-axis is from 0 to 1000, and of the y-axis from -1 to 1E+03.

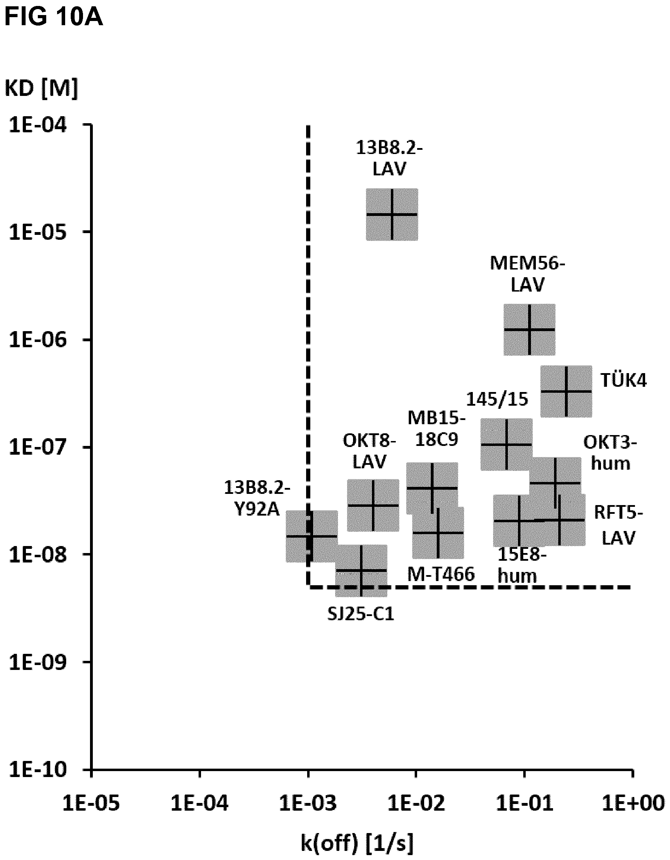

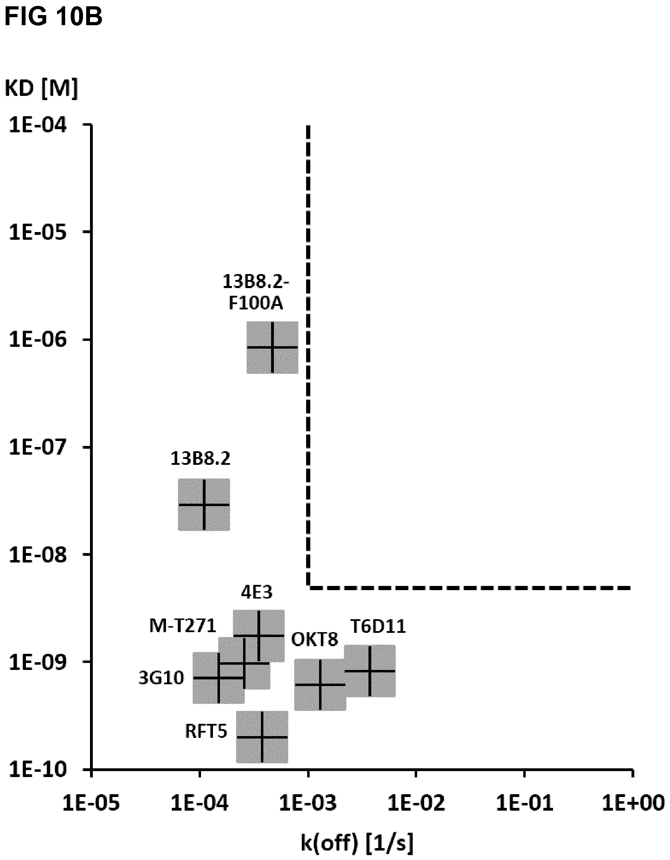

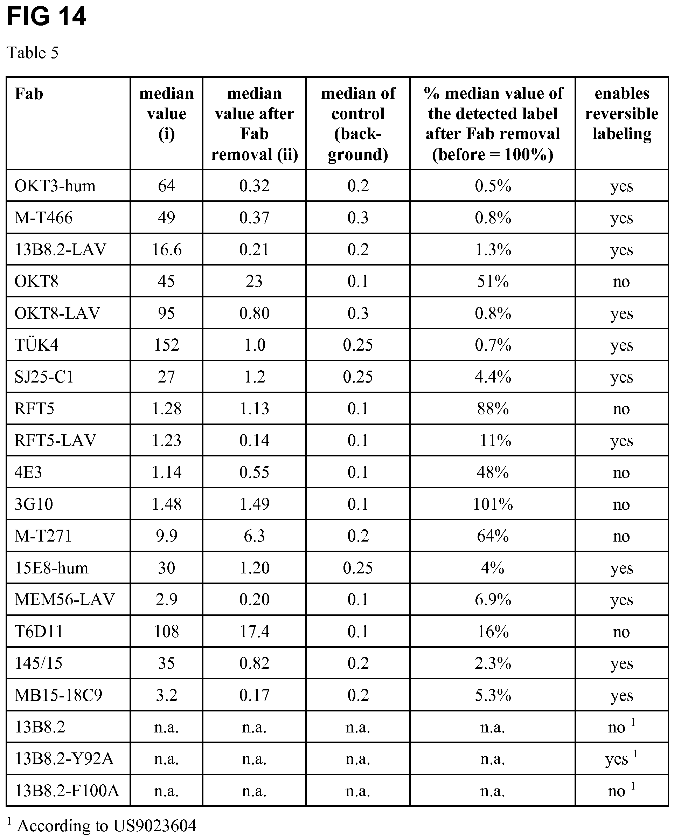

FIG 10: Correlation map of the equilibrium dissociation constant K_{D} [M] vs the dissociation rate constant k(off) [s-1] for Fab fragments, determined by surface plasmon resonance (Biacore™) using recombinant antigens. The crosses for each individual clone mark the approximate error margin of the measured kinetic values. The dashed lines mark the values of 0.5E-08 M for K_{D} and 1E-03 sec-1 for k(off). A: Fabs that reversibly label target antigens, and B: Fabs that do not reversibly label target antigens, as determined by the reduction of the value of the detected label after reversible labeling (as described in Example 8 and compiled in FIG 14 (Table 5)).

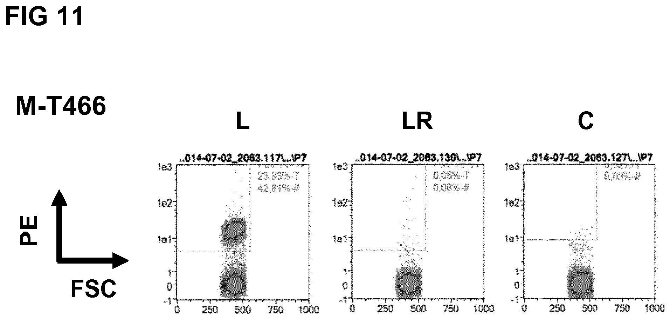

FIG 11: Flow cytometric analysis of reversibly labeled antigens in human PBMCs (peripheral blood mononuclear cells) using a complex of multimerized biotinylated Fabs (anti-CD4 M-T466 as an example) and an anti-biotin antibody that served as multimerization component, directly coupled to PE (phycoerythrin) as detection moiety (anti-Biotin-PE, Miltenyi Biotec). L, labeling of antigen with multimerized Fab; LR, after disruption of the multimerized complex with free biotin and a washing step to remove monomerized and dissociated Fab; C: control staining with anti-Biotin-PE, but without Fab. FSC: forward scatter. The range of the x-axis is from 0 to 1000, and of the y-axis from -1 to 1E+03.

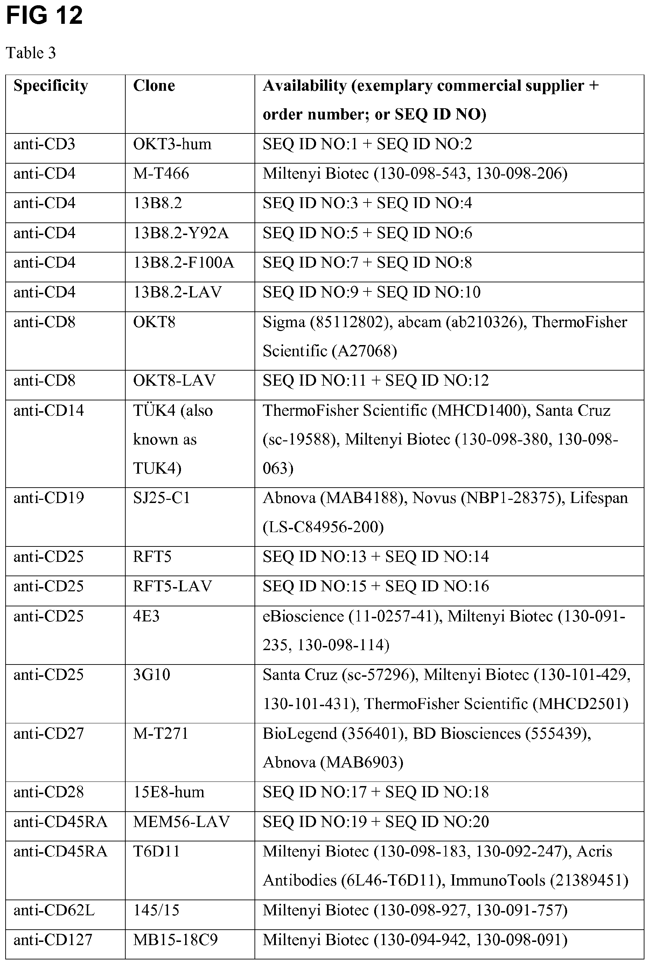

FIG 12: Table 3, availability of used antibodies.

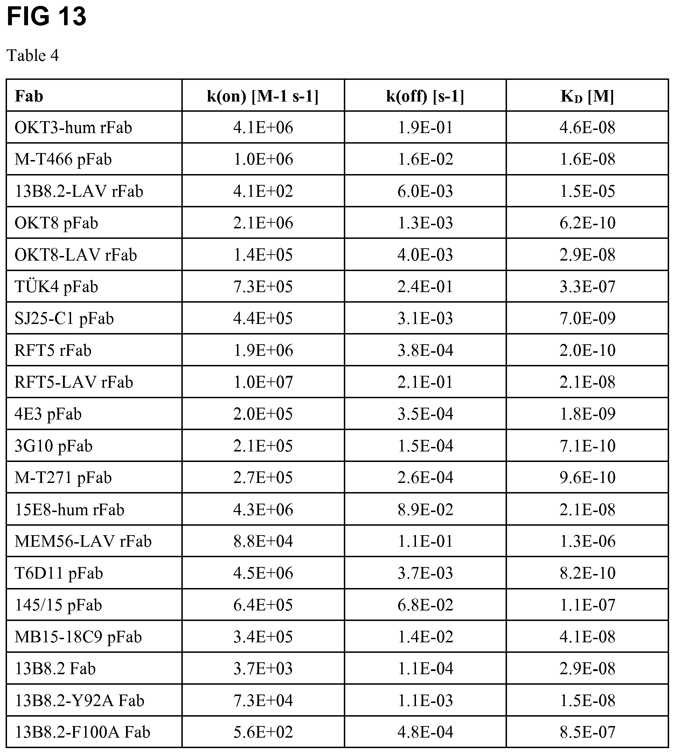

FIG 13: Table 4, kinetic constants of Fab molecules, measured by surface plasmon resonance using recombinant antigens. Values for 13B8.2, 13B8.2-Y92A and 13B8.2-F100A were taken from US7482000. M, molar; s, second.
FIG 14: Table 5, median values of detected label during labeling of antigens on the cell surface by Fabs (i) and after washing to remove Fab molecules (ii). The median value of the antigen / Fab complex (before removal) was set to 100%. A Fab enables reversible labeling of its antigen if the median value of the detected label after reversible labeling is less than 15% of the original median value of the labeled target antigen (i.e. the median is reduced by more than 85% after removal of the Fab).

### Detailed description of the invention

The present invention provides methods for reversible labeling of antigens recognized by monovalent antigen binding molecules having an equilibrium dissociation constant (K_{D}) within a well-defined range and having in addition a dissociation rate constant (k(off)) within a well-defined range.

In a first aspect the invention provides a method for reversible labeling an antigen A of a biological specimen comprising said antigen A with a monovalent antigen binding molecule K comprising a binding site B for specifically binding said antigen A, the method comprising the steps
a) contacting said biological specimen comprising said antigen A with said antigen binding molecule K, thereby generating a complex of A/K,
b) removing the monovalent antigen binding molecule K that is not bound in said complex of A/K, wherein the equilibrium dissociation constant (K_{D}) for the binding of said antigen A and
said monovalent antigen binding molecule K has a value of equal or greater than 0.5E-08 M and wherein the dissociation rate constant (k(off)) of said binding has a value of equal or greater than 1E-03 sec-1.

Said removing in step b) influences the chemical equilibrium of free monovalent antigen binding molecules and monovalent antigen binding molecules bound in the complex.

Said method, wherein the value for K_{D} may be in the range of 0.5E-08 M and 1E-04 M, and wherein the value for k(off) may be in the range of 1E-03 sec-1 and 1E-00 sec-1. Preferred, K_{D} is in the range of 0.5E-08 M and 1E-05 M, and k(off) is in the range of 1E-03 sec-1 and 1E-00 sec-1. More preferred, K_{D} is in the range of 0.5E-08 M and 1E-05 M, and k(off) is in the range of 1E-03 sec-1 and 3E-01 sec-1. Most preferred, K_{D} is in the range of 0.5E-08 M and 1E-06 M, and k(off) is in the range of 1E-03 sec-1 and 3E-01 sec-1.

Said method, wherein at least 85%, at least 90%, at least 95%, at least 96%, at least 97%, at least 98%, at least 99% of the monovalent antigen binding molecule K that is bound in the complex of A/K in step a) is removed in step b).

Said monovalent antigen binding molecule K may be coupled to a detection moiety D, thereby allowing to analyze and/or isolate said complex of A/K between step a) and b) and/or after step b).

The coupling of said monovalent antigen binding molecule K to said detection moiety D may be a covalent or non-covalent coupling.

Said detection moiety may be coupled directly to the monovalent antigen binding molecule, or the coupling may be indirect by the use of a further reagent that couples the detection moiety to the antigen binding molecule.

The detection moiety may be selected from a group consisting of chromophore, fluorescent moiety, phosphorescent moiety, luminescent moiety, light absorbing moiety, radioactive moiety, transition metal isotope mass tag moiety, and magnetic particle.

Said detection moiety may be used to determine the reversibility of labeling, and/or for analyzing the biological specimen, and/or for isolating the biological specimen. For example, the antigen in a biological specimen is contacted with a monovalent antigen binding molecule coupled with a detection moiety. The detectable moiety in the biological specimen is measured by an appropriate method such as flow cytometry for a fluorescent moiety or isotope counting for isotope mass tag moiety (measurement point #1). Afterwards, the monovalent antigen binding molecule coupled with a detection moiety is removed from the antigen within a defined time period. Said time period is preferably not longer than 4 hours, more preferred not longer than 2 hours, more preferred not longer than 1 hour, and most preferred not longer than 30 minutes. The remaining detectable moiety in the biological specimen is measured again by the same method (measurement point #2). The determined values of #1 and #2 are compared. The labeling of the antigen in biological specimens by a monovalent antigen binding molecule is reversible if the value of #2 is less than the value of #1. Preferably, the value of #2 is not more than 15% of the value of #1, more preferred not more than 10% of the value of #1, more preferred not more than 5% of the value of #1, more preferred not more than 3% of the value of #1, and most preferred not more than 1% of the value of #1.

The time period of said contacting of said biological specimen comprising said antigen A with said monovalent antigen binding molecule K may be 1 second to 24 hours, preferentially 1 minutes to 8 hours, more preferentially 1 to 60 minutes and most preferentially 5 to 20 minutes. For removing the monovalent antigen binding molecule in step b) that is not bound in said complex of antigen and monovalent antigen binding molecule, for example washing steps of the antigen / monovalent antigen binding molecule complex in the biological specimen may be performed. By performing a washing step, unbound free monovalent antigen binding molecules are removed from the binding reaction, resulting in a new chemical equilibrium of free monovalent antigen binding molecules and monovalent antigen binding molecules bound in the complex.

Said removing may be performed by at least one washing step.

Several consecutive washing steps may be performed. For example 1 to 10 consecutive washing steps may be performed. Most preferred, 1 to 5 consecutive washing steps may be performed. The duration of a washing step may influence the amount of unbound free monovalent antigen binding molecule that can be removed from the binding reaction. Preferably, the duration of a washing step may be 10 seconds to 60 minutes, more preferred, the duration of a washing step may be 10 seconds to 30 minutes, even more preferred, the duration of a washing step may be 10 seconds to 15 minutes, and most preferred, the duration of a washing step may be 10 seconds to 10 minutes. For example said method may be performed by 1 to 5 consecutive washing steps with a duration of each washing step of 10 seconds to 10 minutes.

In order to maintain the native structure of the antigen, and/or the viability of cells, and/or a structure such as fixed cells, and/or to remove unbound antigen binding molecules, the temperature of step a) "contacting" may be in the range of 0 °C and 50 °C, more preferred in the range of 4 °C and 37 °C, and most preferred in the range of 4 °C and 15 °C; and the temperature of step d) "removing" may be in the range of 0 °C and 50 °C, more preferred in the range of 4 °C and 37 °C, and most preferred in the range of 15 °C and 25 °C.

Said monovalent antigen binding molecule K may comprise monovalent antibodies, Fabs, scFv, and single-domain antibodies. Preferably, said monovalent antigen binding molecule K may be a Fab or a scFv. Most preferred, said monovalent antigen binding molecule K may be a Fab.

In a further aspect the invention provides a method for reversible labeling an antigen A of a biological specimen comprising said antigen A with a monovalent antigen binding molecule K comprising a binding site B for specifically binding said antigen A and at least one binding site Y for coupling to a multimerization component F comprising at least two binding sites G for coupling to said monovalent antigen binding molecule K, wherein said multimerization component F is coupled to a detection moiety D, wherein the assembly of the complex of F/K comprises at least two of said monovalent antigen binding molecule K coupled to said multimerization component F, and wherein F and K are coupled non-covalently via the binding sites G and Y,
the method comprising the steps
a) contacting
   i) said biological specimen comprising said antigen A with said monovalent antigen binding molecule K, thereby generating a complex of A/K, and subsequently contacting said complex of A/K with said multimerization component F, thereby generating a complex of A/K/F; or
   ii) said biological specimen comprising said antigen A with said complex of F/K, wherein said assembly of F/K is performed before step a), thereby generating a complex of A/K/F,
b) analyzing and/or isolating said complex of A/K/F,
c) disrupting the complex of F/K, thereby monomerizing said at least two of said antigen binding molecule K,
d) removing the monovalent antigen binding molecule K that is not bound in said complex of A/K,
wherein the equilibrium dissociation constant (K_{D}) for the binding of said antigen A and said monovalent antigen binding molecule K comprising a binding site B has a value of equal or greater than 0.5E-08 M and wherein the dissociation rate constant (k(off)) of said binding has a value of equal or greater than 1E-03 sec-1.

Said method, wherein at least 85%, at least 90%, at least 95%, at least 96%, at least 97%, at least 98%, at least 99% of the monovalent antigen binding molecule K that is bound in the complex of A/K/F in step a) is removed in step d).

Said removing in step d) influences the chemical equilibrium of free monovalent antigen binding molecules and monovalent antigen binding molecules bound in the complex of A/K. Said method wherein the value for K_{D} may be in the range of 0.5E-08 M and 1E-04 M, and wherein the value for k(off) may be in the range of 1E-03 sec-1 and 1E-00 sec-1.

Preferred, K_{D} is in the range of 0.5E-08 M and 1E-05 M, and k(off) is in the range of 1E-03 sec-1 and 1E-00 sec-1. More preferred, K_{D} is in the range of 0.5E-08 M and 1E-05 M, and k(off) is in the range of 1E-03 sec-1 and 3E-01 sec-1. Most preferred, K_{D} is in the range of 0.5E-08 M and 1E-06 M, and k(off) is in the range of 1E-03 sec-1 and 3E-01 sec-1.

The coupling of said multimerization component F to said detection moiety D may be a covalent or non-covalent coupling.

Said detection moiety may be coupled directly to the multimerization component F, or the coupling may be indirect by the use of a further reagent that couples the detection moiety to the multimerization component F. Preferentially, the non-covalent coupling between the monovalent antigen binding molecule K and the multimerization component F and the non-covalent coupling between the detection moiety D and the multimerization component F are not identical to prevent interference of the two non-covalent couplings.

The detection moiety may be selected from a group consisting of chromophore, fluorescent moiety, phosphorescent moiety, luminescent moiety, light absorbing moiety, radioactive moiety, transition metal isotope mass tag moiety, and magnetic particle.

The time period of said contacting of said biological specimen comprising said antigen A with said monovalent antigen binding molecule K may be 1 second to 24 hours, preferentially 1 minutes to 8 hours, more preferentially 1 to 60 minutes and most preferentially 5 to 20 minutes. The time period of said contacting of said biological specimen comprising the complex of A/K with said multimerization component F may be 1 second to 24 hours, preferentially 1 minutes to 8 hours, more preferentially 1 to 60 minutes and most preferentially 5 to 20 minutes.

The time period of said assembly of F/K (multimerization component/monovalent antigen binding molecule) may be 1 second to 24 hours, preferentially 1 minutes to 8 hours, more preferentially 1 to 60 minutes and most preferentially 5 to 20 minutes.

For removing the monovalent antigen binding molecule in step d) that is not bound in said complex of antigen and monovalent antigen binding molecule, for example washing steps of the antigen / monovalent antigen binding molecule complex in the biological specimen may be performed. By performing a washing step, unbound free monovalent antigen binding molecules are removed from the binding reaction, resulting in a new chemical equilibrium of free monovalent antigen binding molecules and monovalent antigen binding molecules bound in the complex.

Said removing may be performed by at least one washing step.

Several consecutive washing steps may be performed. For example 1 to 10 consecutive washing steps may be performed. Most preferred, 1 to 5 consecutive washing steps may be performed. The duration of a washing step may influence the amount of unbound free monovalent antigen binding molecule that can be removed from the binding reaction. Preferably, the duration of a washing step may be 10 seconds to 60 minutes, more preferred, the duration of a washing step may be 10 seconds to 30 minutes, even more preferred, the duration of a washing step may be 10 seconds to 15 minutes, and most preferred, the duration of a washing step may be 10 seconds to 10 minutes. For example said method may be performed by 1 to 5 consecutive washing steps with a duration of each washing step of 10 seconds to 10 minutes.

In order to maintain the native structure of the antigen, and/or the viability of cells, and/or a structure such as fixed cells, and/or to remove unbound antigen binding molecule, the temperature of step a) "contacting" may be in the range of 0 °C and 50 °C, more preferred in the range of 4 °C and 37 °C, and most preferred in the range of 4 °C and 15 °C; the temperature of step b) "analyzing and/or isolating" may be in the range of 0 °C and 50 °C, more preferred in the range of 4 °C and 37 °C, and most preferred in the range of 4 °C and 15 °C; the temperature of step c) "disrupting" may be in the range of 0 °C and 50 °C, preferably in the range of 4 °C and 37 °C; and the temperature of step d) "removing" may be in the range of 0 °C and 50 °C, more preferred in the range of 4 °C and 37 °C, and most preferred in the range of 15 °C and 25 °C.

Said monovalent antigen binding molecule K may comprise monovalent antibodies, Fabs, scFv, and single-domain antibodies. Preferably, said monovalent antigen binding molecule K may be a Fab or a scFv. Most preferred, said monovalent antigen binding molecule K may be a Fab.

As described, said multimerization component F may be coupled non-covalently to the monovalent antigen binding molecule by the interaction of said binding sites G of the multimerization component F with at least two monovalent antigen binding molecules K each comprising at least one binding site Y. As a result, the monovalent antigen binding molecules become multimerized, leading to multivalent binding and an increased avidity of the binding between antigen binding molecules and antigen. For contacting an antigen A in biological specimen, either the antigen A is contacted with the monovalent antigen binding molecule K, forming a complex of antigen A and monovalent antigen binding molecule K, and afterwards said complex is contacted with said multimerization component F. Or monovalent antigen binding molecule K and multimerization component F are contacted to form a complex comprising multimerized monovalent antigen binding molecules, and said complex is then contacted with an antigen. For removal of the detection moiety and the multimerized monovalent antigen binding molecules bound to the antigen, the complex of multimerization component, monovalent antigen binding molecules, and antigen may be disrupted in order to monomerize the monovalent antigen binding molecules.

Disruption may take place at the binding between multimerization component and monovalent antigen binding molecules. An example for a disruption may be the addition of a reagent competing with the multimerization component and/or the monovalent antigen binding molecules in the case of a non-covalent binding. In another example, the multimerization component itself is disrupted in order to monomerize antigen binding molecules. Said disruption of the multimerization component itself may be conducted, for example, by enzymatic digestion, chemical cleavage, and/or mechanical shearing. Preferably, the disruption may be conducted enzymatically and/or chemically. The multimerization component may comprise an enzymatically degradable material. Methods to monomerize antigen binding molecules after multimerization with a multimerization component may be combined, for example a combination of adding a competitor of the binding between antigen binding molecule and multimerization component plus enzymatic digestion or chemical cleavage of the multimerization component.

Therefore, said method may be performed, wherein said disruption is performed by
(i) contacting said complex of F/K in step c) with a molecule that competes with F for the binding to K or that competes with K for the binding to F, thereby replacing F or K in the complex of F/K, respectively; or
(ii) chemical or enzymatic disruption within the multimerization component F; or
(iii) a combination of (i) and (ii).

In another example, the conformation of the multimerization component and/or the antigen binding molecule may be altered in order to disrupt the binding between multimerization component and monovalent antigen binding molecules. Said conformational alteration may be induced by, for example, changes in temperature, pH, and salt concentrations. In a more specific approach, the antigen binding molecule may comprise a component that is capable to change its conformation upon a stimulus. For example, calmodulin or a variant thereof may be inserted in the antigen binding molecule, and adding or removing Ca²⁺ induces a conformational change of the multimerization component due to the conformational change of calmodulin. The conformational change of the multimerization component disrupts the binding of at least some the monovalent antigen binding molecules to the multimerization component, and as a result the monovalent antigen binding molecules become monomerized.
Methods to monomerize antigen binding molecules after multimerization with a multimerization component may be combined, for example a combination of adding a competitor of the binding between monovalent antigen binding molecule and multimerization component plus enzymatic digestion of the multimerization component.

In another aspect the invention provides a method for reversible labeling an antigen A of a biological specimen comprising said antigen A with a monovalent antigen binding molecule K comprising a binding site B for specifically binding said antigen A, wherein at least two of said monovalent antigen binding molecule K are coupled to a multimerization component F, thereby generating a complex of F/K, wherein F and K are coupled covalently, wherein said multimerization component F is coupled to a detection moiety D, the method comprising the steps
a) contacting said biological specimen comprising said antigen A with said complex of F/K, thereby generating a complex of A/K/F,
b) analyzing and/or isolating said complex of A/K/F,
c) disrupting the complex of F/K, thereby monomerizing said at least two of said antigen binding molecule K,
d) removing the monovalent antigen binding molecule K that is not bound in said complex of A/K,
wherein the equilibrium dissociation constant (K_{D}) for the binding of said antigen A and said antigen binding molecule K comprising a binding site B has a value of equal or greater than 0.5E-08 M and wherein the dissociation rate constant (k(off)) of said binding has a value of equal or greater than 1E-03 sec-1.

Said method, wherein at least 85%, at least 90%, at least 95%, at least 96%, at least 97%, at least 98%, at least 99% of the monovalent antigen binding molecule K that is bound in the complex of A/K/F in step a) is removed in step d).

Said removing in step d) influences the chemical equilibrium of free monovalent antigen binding molecules and monovalent antigen binding molecules bound in the complex of A/K.

Said method wherein the value for K_{D} may be in the range of 0.5E-08 M and 1E-04 M, and wherein the value for k(off) may be in the range of 1E-03 sec-1 and 1E-00 sec-1. Preferred, K_{D} is in the range of 0.5E-08 M and 1E-05 M, and k(off) is in the range of 1E-03 sec-1 and 1E-00 sec-1. More preferred, K_{D} is in the range of 0.5E-08 M and 1E-05 M, and k(off) is in the range of 1E-03 sec-1 and 3E-01 sec-1. Most preferred, K_{D} is in the range of 0.5E-08 M and 1E-06 M, and k(off) is in the range of 1E-03 sec-1 and 3E-01 sec-1.

The coupling of said multimerization component F to said detection moiety D may be a covalent or non-covalent coupling.

Said detection moiety may be coupled directly to the multimerization component F, or the coupling may be indirect by the use of at least one further reagent that couples the detection moiety to the multimerization component F.

The detection moiety may be selected from a group consisting of chromophore, fluorescent moiety, phosphorescent moiety, luminescent moiety, light absorbing moiety, radioactive moiety, transition metal isotope mass tag moiety, and magnetic particle.

The time period of said contacting of said biological specimen comprising said antigen A with said complex of F/K (multimerization component/monovalent antigen binding molecule) may be 1 second to 24 hours, preferentially 1 minutes to 8 hours, more preferentially 1 to 60 minutes and most preferentially 5 to 20 minutes.

For removing the monovalent antigen binding molecule in step d) that is not bound in said complex of antigen and monovalent antigen binding molecule, for example washing steps of the antigen / monovalent antigen binding molecule complex in the biological specimen can be performed. Performing a washing step, unbound free monovalent antigen binding molecules are removed from the binding reaction, resulting in a new chemical equilibrium of free monovalent antigen binding molecules and monovalent antigen binding molecules bound in the complex.

Said removing may be performed by at least one washing step.

Several consecutive washing steps may be performed. For example 1 to 10 consecutive washing steps may be performed. Most preferred, 1 to 5 consecutive washing steps may be performed. The duration of a washing step may influence the amount of unbound free monovalent antigen binding molecule that can be removed from the binding reaction. Preferably, the duration of a washing step may be 10 seconds to 60 minutes, more preferred, the duration of a washing step may be 10 seconds to 30 minutes, even more preferred, the duration of a washing step may be 10 seconds to 15 minutes, and most preferred, the duration of a washing step may be 10 seconds to 10 minutes. For example said method may be performed by 1 to 5 consecutive washing steps with a duration of each washing step of 10 seconds to 10 minutes.

In order to maintain the native structure of the antigen, and/or the viability of cells, and/or a structure such as fixed cells, and/or to remove unbound antigen binding molecule, the temperature of step a) "contacting" may be in the range of 0 °C and 50 °C, more preferred in the range of 4 °C and 37 °C, and most preferred in the range of 4 °C and 15 °C; the temperature of step b) "analyzing and/or isolating" may be in the range of 0 °C and 50 °C, more preferred in the range of 4 °C and 37 °C, and most preferred in the range of 4 °C and 15 °C; the temperature of step c) "disrupting" may be in the range of 0 °C and 50 °C, preferably in the range of 4 °C and 37 °C; and the temperature of step d) "removing" may be in the range of 0 °C and 50 °C, more preferred in the range of 4 °C and 37 °C, and most preferred in the range of 15 °C and 25 °C.

Said monovalent antigen binding molecule K may comprise monovalent antibodies, Fabs, scFv, and single-domain antibodies. Preferably, said monovalent antigen binding molecule K may be a Fab or a scFv. Most preferred, said monovalent antigen binding molecule K may be a Fab.

As described, said multimerization component F may be coupled covalently to the monovalent antigen binding molecule K by the interaction of the multimerization component F with at least two monovalent antigen binding molecules. As a result, the monovalent antigen binding molecules K become multimerized, leading to multivalent binding and an increased avidity of the binding between antigen binding molecules K and antigen A. For contacting an antigen in biological specimen, the antigen is contacted with the complex K/F of antigen binding molecules and multimerization component, forming a complex A/K/F of antigen, antigen binding molecule, and multimerization component. For removal of the detection moiety D and the multimerized antigen binding molecules K bound to the antigen, the complex of multimerization component F, antigen binding molecules K, and antigen A may be disrupted in order to monomerize the antigen binding molecules K. Disruption may take place at the binding interaction between multimerization component F and antigen binding molecules K. Examples for a disruption comprises enzymatic (e.g. site-specific proteolysis) and chemical (e.g. photochemical) cleavage of a covalent binding between multimerization component and antigen binding molecules. An enzymatically degradable spacer may be used for binding between multimerization component and antigen binding molecules that can be cleaved by a specific enzyme. In another example, the multimerization component itself is disrupted in order to monomerize antigen binding molecules. Said disruption of the multimerization component itself may be conducted, for example, by enzymatic digestion, chemical cleavage, and/or mechanical shearing. Preferably, the disruption may be conducted enzymatically and/or chemically. The multimerization component may comprise an enzymatically degradable material. Methods to monomerize antigen binding molecules after multimerization with a multimerization component may be combined, for example a combination of enzymatic or chemical cleavage of the covalent binding between antigen binding molecule and multimerization component plus enzymatic digestion or chemical cleavage of the multimerization component.

Therefore, said method may be performed wherein said disruption is performed by
(i) chemical or enzymatic disruption within the multimerization component F; or
(ii) chemical or enzymatic disruption of the covalent bound of K/F; or
(iii) a combination of (i) and (ii).

In another aspect, the invention provides monomeric antigen binding molecules against several antigens. Some of the antigen binding molecules have kinetic parameters (K_{D}, k(off)) that enable a reversible binding to and therefore reversible labeling of antigens in biological specimen. These molecules can be derived by hybridoma techniques, recombinant methods as well as by enzymatic digestion of antibodies. Preferably said antigen binding molecules are derived by recombinant methods. Said antigen binding molecules comprise a binding site for specifically binding the antigen.

In a further aspect the invention provides a method for identification of monovalent antigen binding molecules that enable the reversible labeling of antigen, the method comprising the step of analyzing the association and dissociation reaction between said antigen and said monovalent antigen binding molecule, thereby determining the equilibrium dissociation constant (K_{D}) and the dissociation rate constant (k(off)), and the association rate constant k(on)), wherein a K_{D} of equal or greater than 0.5E-08 M and a (k(off)) of equal or greater than 1E-03 sec-1 is essential for a monovalent antigen binding molecule enabling the reversible labeling of an antigen. Said method wherein the value for K_{D} may be in the range of 0.5E-08 M and 1E-04 M, and wherein the value for k(off) may be in the range of 1E-03 sec-1 and 1E-00 sec-1. Preferred, K_{D} is in the range of 0.5E-08 M and 1E-05 M, and k(off) is in the range of 1E-03 sec-1 and 1E-00 sec-1. More preferred, K_{D} is in the range of 0.5E-08 M and 1E-05 M, and k(off) is in the range of 1E-03 sec-1 and 3E-01 sec-1. Most preferred, K_{D} is in the range of 0.5E-08 M and 1E-06 M, and k(off) is in the range of 1E-03 sec-1 and 3E-01 sec-1.

Said method, wherein said step of analyzing the association and dissociation reaction may be performed by, for example, surface plasmon resonance (SPR), quartz crystal microbalance (QCM), kinetic exclusion assay (KinExA), and Bio-layer interferometry (BLI). The antigen used in said step of analyzing the association and dissociation reaction may be isolated and may comprise at least the functional epitope bound by the antigen binding molecule. Preferably, the antigen comprises the full-length protein or alternatively, in the case of membrane bound antigens, the extracellular domain. The antigen may be derived by recombinant methods as well as by isolation from a natural source.

For removal, enrichment, isolation, or selection of antigen(s) in a biological specimen in principle any sorting technology can be used. This includes for example affinity chromatography or any other antibody-dependent separation technique known in the art. Any ligand-dependent separation technique known in the art may be used.

An especially potent sorting technology is magnetic cell sorting. Methods to separate antigens, e.g. expressed on the surface of cells, magnetically are commercially available e.g. from several suppliers. In a preferred embodiment for enriching, sorting and/or detecting antigen in a biological specimen according to the present invention monoclonal antibodies or antigen binding fragments thereof are used in conjunction with colloidal superparamagnetic microparticles having an organic coating by e.g. polysaccharides (Magnetic-activated cell sorting (MACS®) technology (Miltenyi Biotec, Bergisch Gladbach, Germany)).

Another sorting technology uses flow cytometry. Flow cytometry is a laser- or impedance-based, biophysical technology employed e.g. in cell sorting and biomarker detection by suspending e.g. cells in a stream of fluid and passing them by an electronic detection apparatus. It allows simultaneous multiparametric analysis of the physical and chemical characteristics of up to thousands of particles per second. Fluorescence-activated cell sorting (FACS) is a specialized type of flow cytometry. It provides a method for sorting a heterogeneous mixture of biological cells into two or more containers, one cell at a time, based upon the specific light scattering and fluorescent characteristics of each cell.

The methods as disclosed in the present invention does not require the knowledge of the sequence of a monovalent antigen binding molecule. Rather, the kinetic values (K_{D}, k(off)) of the binding of said monovalent antigen binding molecule to the antigen are sufficient to determine whether the monovalent antigen binding molecules enable the reversible labeling of an antigen in a biological specimen. Said kinetic values (K_{D}, k(off)) can be determined, for example, by surface plasmon resonance (SPR, as shown in example 6), quartz crystal microbalance (QCM), kinetic exclusion assay (KinExA), and Bio-layer interferometry (BLI). As an example, hybdridoma antibodies with unknown protein sequence can be used. Those antibodies, against a huge number of antigens, are readily available from several commercial sources. A monovalent Fab can be prepared from a hybridoma antibody, as described in example 1. Antigens are also readily available from several commercial sources (e.g. as recombinant proteins) and can be used for the kinetic measurement in combination with the Fab. The method of the present invention is not limited to certain antigens or monovalent antigen binding molecules, but can be used with any antigen and monovalent antigen binding molecule.

### Definitions

Unless defined otherwise, technical and scientific terms used herein have the same meaning as commonly understood by one of ordinary skill in the art to which this invention belongs.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, immunoadhesins and antibody-immunoadhesin chimeras, that specifically recognize (i.e. bind) a target antigen. "Antibody fragments" or "antigen binding fragments of an antibody" comprise a portion of a full length antibody that is able to specifically bind the antigen or at least the antigen binding site thereof. Examples of antibody fragments include Fab (fragment antigen binding), Fab', F(ab')2, scFv (single chain fragment variable), di-scFv, single-domain antibodies Fab (sdAb), diabodies, nanobodies, single-chain antibody molecules, and multispecific antibodies formed from antibody fragments.

As used herein, the term "antigen" is intended to include substances that evoke the production of one or more antibodies. Each antibody binds to a specific antigen by way of an interaction similar to the fit between a lock and a key. The substance may be from the external environment or formed within the body. The term "antigen" comprises, but is not limited to, proteins, peptides, polypeptides, oligopeptides, lipids, carbohydrates, haptens, vitamins, synthetic molecules and combinations thereof, for example a glycosylated protein or a glycolipid. An antigen may be on the cell surface, inside the cell, outside of a cell (for example in the extracellular matrix), or isolated. Preferentially, an antigen is on the cell surface, inside a cell, or outside of a cell. Preferentially, antigens are mammalian antigens.

The term "monovalent antigen binding molecule" as used herein refers to a molecule that binds specifically to an antigen via its monovalent antigen binding site. The term "binding site" in the context of an antigen binding molecule as used herein refers to a locally defined site of an antigen binding molecule by which the antigen binding molecule and the antigen interact specifically by non-covalent binding, for example one amino acid residue or a chemical group of the antigen binding molecule that interacts by non-covalent binding with one amino acid residue or a chemical group of the antigen. In the case of an antibody, the antigen binding site is usually created by some amino acid residues of the six complementary determining regions. Examples for antigen binding molecules comprise antibodies, antibody fragments such as Fab and scFv, single-domain antibodies, antibody mimetics such as Anticalins, DARPins and monobodies, receptors (wherein the ligand is the antigen), peptide/MHC-complexes targeting TCR molecules, small molecules, and artificial engineered binding molecules, e.g., peptides or aptamers which target, for example, cell surface molecules.

A "monovalent" binding of an antigen binding molecule to an antigen refers to a binding interaction wherein the antigen is bound by one binding site of an antigen binding molecule. Although antigen binding molecules with more than one binding site such as antibodies may be capable to bind an antigen in a monovalent way under certain circumstances, the term "monovalent binding" as used herein refers to antigen binding molecules that have only a single binding site for the antigen). A "monovalent antigen binding molecule" therefore has a single binding site for the antigen. Due to the nature of a monovalent binding, no avidity effect takes place. Examples for monovalent antigen binding molecules comprise Fab, scFv, single chain antibodies, single-domain antibodies, and monovalent antibody mimetics.

A "multivalent" binding of an antigen binding molecule to an antigen refers to a binding interaction wherein the antigen is bound by at least two binding sites of (an) antigen binding molecule(s) at the same time. The binding sites can be located on the same antigen binding molecule (for example, an antibody) or on separated antigen binding molecules (for example, Fab or scFv molecules). In the latter case, multivalent binding of monovalent antigen binding molecules may be achieved for example by multimerization of the antigen binding molecules using a multimerization component that brings at least two monovalent antigen binding molecule in steric proximity in order to force binding of both monovalent antigen binding molecules to antigen at the same time. Due to the multivalent binding, an avidity effect takes place.

"Multimerization" (of monovalent antigen binding molecules) as used herein refers to a method by which monovalent antigen binding molecules are bound or coupled directly or indirectly to each other in order to enable a bivalent or multivalent non-covalent binding of the antigen and to enable or increase avidity of this binding interaction. One example comprises biotinylated Fab molecules that are multimerized by streptavidin, wherein a streptavidin molecule is bound to at least two Fab molecules. In another example, monomeric scFv molecules are coupled to a magnetic particle. In another example, an antibody can be regarded as the result of a multimerization of two monomeric Fab molecules.

"Monomerization" (of multimerized monovalent antigen binding molecules) as used herein refers to a method by which monovalent antigen binding molecules that are bound or coupled directly or indirectly to each other and therefore exhibit a bi- or multivalent non-covalent binding towards the antigen are separated from each other by disruption of said direct or indirect binding or coupling to each other in order to eliminate the multivalency of antigen binding by the antigen binding molecules. One example comprises a complex of Fab molecules comprising a biotin mimicking peptide per Fab that are multimerized with streptavidin, wherein one streptavidin molecule is bound to at least two Fab molecules via said biotin mimicking peptides, and wherein the Fab molecules are separated from each other (monomerized) by the addition of free biotin that competes with the biotin mimicking peptides for the binding to streptavidin. In another example, a complex of monomeric scFv molecules coupled to particles, wherein at least two scFv molecules are coupled to one particle, is disrupted by the addition of an enzyme that degrades at least the outer shell of the particle where the scFv molecules are coupled to. In another example, enzymatic cleavage of an antibody specific for an antigen with papain can be regarded as a monomerization, because two monovalently binding Fab fragments are generated. The terms "avidity" and "avidity effect" have an interchangeable meaning and refer to the accumulated strength of multiple affinities of individual non-covalent binding interactions, such as between a protein receptor and its ligand, or an antibody and its antigen. Each antibody has at least two antigen binding sites, therefore antibodies are bivalent to multivalent. For example IgM is said to have low affinity but high avidity because it has 10 weak binding sites for antigen as opposed to the 2 stronger binding sites of IgG, IgE and IgD with higher single binding affinities.

The term "covalent" with respect to a binding or coupling interaction between two molecules or two sites in the same molecule as used herein refers to a chemical covalent bond. This comprises the covalent binding of two proteins to each other (for example by a disulfide bridge), the covalent binding of a protein to a chemical group of another molecule (for example by a disulfide or an amide bond), and the covalent binding of two chemical elements or groups (for example by a disulfide or an amide bond). A covalent bond can be a single, a double and a triple bond.

The term "non-covalent" with respect to a binding or coupling interaction between two molecules or two sites in the same molecule as used herein refers to all non-covalent interactions, for example hydrogen bonds, halogen bonds, ionic interactions, hydrophobic effects, π-effects, and van der Waals forces.

The term "binding" as used herein generally refers to all covalent and non-covalent interactions between two molecules/entities or two sites on a molecule/entity. In particular, the term is preferentially used herein for the interaction between the antigen binding molecule K and the antigen. Other examples for bindings comprise the generation of a chemical bond between a fluorochrome and a protein, the interaction between biotin and streptavidin or an anti-biotin antibody, and generation of a chemical bond between a polysaccharide and a protein or a chemical moiety.

The term "coupling" is used herein preferentially for the binding of antigen binding molecule K to the detection moiety D, the binding of antigen binding molecule K to the multimerization component F, and the binding of the multimerization component F to the detection moiety D. Said coupling of these molecules/entities can be direct or indirect.

For coupling, a spacer may be used, resulting in an indirect coupling. In case of a covalent bound between a molecule and the spacer, a direct reaction of an activated group either on the molecule or on the spacer with a functional group on either the spacer or on the molecule or via a heterobifunctional linker molecule, which is firstly reacted with one and secondly reacted with the other binding partner is possible. For example, a large number of heterobifunctional compounds are available for linking to entities. Illustrative entities include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-y-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, succinimidyl-[(N-maleimidopropionamido) polyethyleneglycol] esters (NHS-PEG-MAL), and succinimidyl 4-[N-maleimidomethyl]cyclohexane-1-carboxylate. A preferred linking group is 3-(2-pyridyldithio)propionic acid N-hydroxysuccinimide ester (SPDP), or 4-(N-maleimidomethyl)-cyclohexane-1-carboxylic acid N-hydroxysuccinimide ester (SMCC).

The term "conjugate" as used herein refers to an antigen binding molecule coupled to a detection moiety, for example anti-CD4-PE, an anti-CD4 antibody coupled to phycoerythrin (PE).

Methods for covalently or non-covalently coupling are known by persons skilled in the art. In case of a covalent bound between the detection moiety and the spacer, a direct reaction of an activated group either on the detection moiety or on the spacer with a functional group on either the spacer or on the detection moiety or via a heterobifunctional linker molecule, which is firstly reacted with one and secondly reacted with the other binding partner is possible.

The term "indirect binding" with respect to the binding of molecules and/or moieties refers to a covalent or non-covalent binding of two partners such as two molecules, or one molecule and one moiety, or two moieties, to each other, wherein at least a third reagent is used to generate the binding of the two partners to each other. The binding of said third reagent to a molecule and or moiety may be covalent or non-covalent. Said third reagent may also bind covalently or non-covalently to a fourth reagent that itself is also used to generate the binding of the two partners to each other by direct binding of the third and fourth reagent to each other. An example for indirect binding of the detection moiety to the monovalent antigen binding molecule comprises a biotin molecule ("third reagent") coupled to the monovalent antigen binding molecule and a detection moiety coupled to avidin, streptavidin, or an anti-biotin antibody ("fourth reagent"). In another example for indirect binding, the monovalent antigen binding molecule comprises a poly-histidine sequence, and the detection moiety is coupled to an anti-poly-histidine antibody ("third reagent"). Another example of indirect binding makes use of a spacer ("third reagent") that is bound covalently or non-covalently to the monovalent antigen binding molecules and to the detection moiety. In another example for indirect binding, an antibody binds to its antigen in a Ca²⁺ ("third reagent") dependent manner, i.e. in the presence of Ca²⁺ both molecules bind to each other and to Ca²⁺, while in the absence of Ca²⁺ there is no specific binding between antibody and antigen.

The term "direct binding" with respect to the binding of molecules and/or moieties refers to a covalent or non-covalent binding of two partners such as two molecules, or one molecule and one moiety, or two moieties, to each other, wherein no third reagent is required to generate the binding of the two partners to each other. For example, in the case of direct binding of two proteins to each other, a covalent binding may be generated by forming a disulfide bond between a cysteine residue of both proteins. The non-covalent binding of an antibody or a Fab molecule to an antigen is another example for direct binding.

The terms "specifically binds to" or "specific for" with respect to the antigen binding site of an antigen binding molecule refer to an antigen binding site which recognizes and binds to a specific antigen, i.e. CD3 in the case of an anti-CD3 antibody or fragment thereof, but does not substantially recognize or bind other antigens in a sample. An antigen binding site that binds specifically to an antigen from one species may bind also to that antigen from another species. This cross-species reactivity is not contrary to the definition of that antigen binding site as specific. An antigen binding site that specifically binds to an antigen may bind also to different allelic forms of the antigen (allelic variants, splice variants, isoforms etc.). This cross reactivity is not contrary to the definition of that antigen binding site as specific.

The antigen binding molecules according to the invention may contain amino acid substitutions, insertions and deletions. For example, a poly-histidine sequence may be inserted or added to any Fab fragment chain which allows for purification using affinity chromatography and/or immunological detection using anti-histidine antibodies.

The antibody according to the invention is preferably characterized in that the constant domains are of human origin. Such constant domains are well known in the art and described, e.g. by Kabat.

The terms "equilibrium dissociation constant" and "affinity constant" (often truncated as "affinity") as used herein have an interchangeable meaning and describe the kinetic constant K_{D} [M] of two binding partners A and B (such as an antibody and its antigen or a receptor and its ligand) that is defined by the formula K_{D} = k(off)/k(on), wherein k(off) is the "dissociation rate constant" [s-1] and k(on) is the "association rate constant" [M-1 s-1]. These constants K_{D}, k(on) and k(off) can be determined by common methods known in the art (e.g., Biacore™ measurement). In the present application, the values of these kinetic constants refer to their determination under "standard conditions", i.e. a controlled temperature of 25 °C and atmospheric pressure of approximately 1.013 bar.

The term "biological specimen" as used herein may be a cell or cells, preferentially in solution, tissue(s), an extracellular matrix or matrices, extracellular vesicle(s), organelle(s), whole blood, plasma, urine, feces, saliva, viral particles, or a combination thereof. Generally, a "biological specimen" as used herein means a sample of biological source comprising an antigen that may be capable of being bound by a monovalent antigen binding molecule, wherein said monovalent antigen binding molecule may be subsequently removed from the antigen and from the biological specimen as disclosed herein.

The term "isolated" means altered or removed from the natural state. For example an isolated population of cells means an enrichment of such cells and separation from other cells which are normally associated in their naturally occurring state with said isolated cells. An isolated population of cells means a population of substantially purified cells which are a more homogenous population of cells than found in nature. Preferably the enriched cell population comprises at least about 90% of the selected cell type. In particular aspects, the cell population comprises at least about 90%, 91%, 92%, 93%, 94%, 95%, 96%, 97%, 98%, 99%, or even 100% of the selected cell type. For example, a nucleic acid or a peptide naturally present in a living animal is not "isolated", but the same nucleic acid or peptide partially or completely separated from the coexisting materials of its natural state is "isolated". An isolated nucleic acid or protein can also exist in a non-native environment such as, for example, a host cell.

The term "particle" as used herein refers to a solid phase such as colloidal particles, microspheres, nanoparticles, or beads. Methods for generation of such particles are well known in the field of the art. The particles may be magnetic particles. The particles may be in a solution or suspension or they may be in a lyophilized state prior to use in the present invention. The lyophilized particle is then reconstituted in convenient buffer before contacting the sample to be processed regarding the present invention.

The term "magnetic" in "magnetic particle" as used herein refers to all subtypes of magnetic particles which could be prepared with methods well known to the skilled person in the art, especially ferromagnetic particles, superparamagnetic particles and paramagnetic particles. The terms "detection moiety", "detectable label", and "detected label" as used herein have an interchangeable meaning and refer to any moiety possessing a property or function which can be used for detection purposes. Said detection moiety may be attached, by a covalent or non-covalent coupling, directly or indirectly to another molecule, preferentially to an antigen binding molecule and/or a multimerization component. Said detection moiety may be used, for example, to detect, trace, separate, isolate, deplete, analyze, or quantify the labeled molecule and thereby the antigen-comprising biological specimen. For example stronger labeled cells, weaker labeled cells, and non-labeled cells may be discriminated and/or isolated, e.g. via flow cytometry, fluorescent cell sorting, magnetic cell sorting, or fluorescence microscopy.

The detection moiety may be selected from a group consisting of chromophore, fluorescent moiety, phosphorescent moiety, luminescent moiety, light absorbing moiety, radioactive moiety, transition metal isotope mass tag moiety, and magnetic particle. Preferably, said detection moiety may be a chromophore, fluorescent moiety, phosphorescent moiety, luminescent moiety, light absorbing moiety, or particle. More preferred, said detection moiety may be a fluorescent moiety, phosphorescent moiety, luminescent moiety, light absorbing moiety, or magnetic particle. Most preferred, said detection moiety may be a fluorescent moiety, luminescent moiety, light absorbing moiety, or magnetic particle. Detection moieties may be combined, for example a magnetic particle may be used in combination with a fluorescent moiety. In this case, it is sufficient when the monovalent antigen binding molecule is coupled to at least one of the combined detection moieties (e.g. the monovalent antigen binding molecule is coupled to the magnetic particle, and the magnetic particle is coupled to the fluorescent moiety).

The method and equipment to detect the detection moiety and to quantify its signal is determined by the detection moiety.

Useful fluorescent moieties might be protein-based, such as phycobiliproteins, polymeric, such as polyfluorenes, small organic molecule dyes, such as xanthenes, like fluorescein, or rhodamines, cyanines, oxazines, coumarins, acridines, oxadiazoles, pyrenes, pyrromethenes, or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties. Another group of photoluminescent detection moieties are phosphorescent moieties with time-delayed emission of light after excitation. Phosphorescent moieties include metallo-organic complexes, such as Pd, Pt, Tb, Eu complexes, or nanoparticles with incorporated phosphorescent pigments such as lanthanide doped SrAl₂O₄. Radioactive moieties may be in the form of radioisotope labeling by exchanging nonradioactive isotopes for their radioactive counterparts, such as tritium, 32P, 35S or 14C, or introducing covalently bound labels, such as 125I, which is bound to tyrosine, 18F within fluorodeoxyglucose, or metallo-organic complexes, i.e. 99Tc-DTPA. A detection moiety may be capable of causing chemiluminescence, i.e. horseradish peroxidase label in the presence of luminol, or may be capable of absorbing of UV, visible light, or NIR radiation. Suitable light-absorbing detection moieties are light absorbing dyes without fluorescence emission, such as small organic molecule quencher dyes like N-aryl rhodamines, azo dyes, and stilbenes. A light-absorbing detection moiety may be irradiated by pulsed laser light, generating an photoacoustic signal. An isotope mass tag moiety may be detected by mass spectrometric detection of a transition metal isotope. Transition metal isotope mass tag labels might be introduced as covalently bound metallo-organic complexes or nanoparticle component. Known in the art are isotope tags of lanthanides and adjacent late transition elements.

The terms "reversible labeling" and "reversibly labeled" have an interchangeable meaning and refer to the feature of a monovalent antigen binding molecule that it is able to bind to an antigen (in a monomeric and/or multimerized form, i.e. monovalently and or multivalently binding) and subsequently can be removed from said antigen, wherein the measured value of the detected label after reversible labeling is less than the measured value of the labeled target antigen. The measurement method and the kind and absolute numbers of measured values of the label depends on the nature of the label. For example the labeling is reversible when in a method that is able to detect a label and to determine a quantitative value for the label signal, such as flow cytometry, fluorescence microscopy and ELISA in the case of a fluorescent label, a quantitative value of the antigen after removal of the antigen binding molecule and the label after at least one washing step of the antigen to remove unbound monovalent antigen binding molecule (measurement point #2) has decreased to less than 15%, 10%, 5%, 4%, 3%, 2%, or 1% of the original quantitative value (measurement point #1). The removal of unbound monovalent antigen binding molecule may be performed in a defined time period. Said time period is preferably not longer than 4 hours, more preferred not longer than 2 hours, more preferred not longer than 1 hour, and most preferred not longer than 30 minutes. Preferably said quantitative value of the labeled target antigen (measurement point #2) has decreased to less than 15%, more preferably to less than 10%, more preferably to less than 5%, more preferably to less than 4%, more preferably to less than 3%, more preferably to less than 2%, and most preferably to less than 1%, compared to the original quantitative value (measurement point #1). The signal of the detected label after removal of the antigen binding molecule (measurement point #2) may be compared to the background signal to decide whether the signal after removal of the antigen binding molecule is still a signal for a specific labeling (i.e. no reversible labeling) or not (i.e. reversible labeling). A ratio of more than 5:1 of measurement point #2 vs. background signal may be indicative that the signal after removal of the antigen binding molecule is still a specific labeling signal when using flow cytometry. Accordingly, a ratio of 5:1 or less of measurement point #2 vs. background signal may be indicative that the antigen binding molecules is completely removed. Said ratio may be different for various detection methods.

The single letters A (antigen), K (antigen binding molecule), F (multimerization component), B (antigen binding site), Y (multimerization component binding site), G (antigen binding molecule binding site), and D (detection moiety) were chosen arbitrarily and serve merely as auxiliary means for a better understanding of the complexes generated as disclosed herein. The order of mention of the single letters within a complex has no specific meaning. For example, the complex A/K is identical to K/A, and means the complex of antigen and antigen binding molecule, and the complex A/F/K is identical to A/K/F, and means the complex of antigen, antigen binding molecule, and multimerization component.

The term "range of <value 1> and < value 2>" as used herein is meant to include all values between <value 1> and <value 2> and also includes <value 1> and <value 2>. For example, a "range of 0.5E-08 M and 1E-05 M" includes the values 0.5E-08 M and 1E-05 M.

Buffers for use in methods as disclosed herein comprise, for example, phosphate, Tris, Hepes, MES, or MOPS, salts such as NaCl, KCl, or MgCl2, chelators such as EDTA or EGTA, and detergents such as Tween-20, Tween-80, Pluronic, or Triton X-100, stabilizers such as BSA (bovine serum albumin) or HSA (human serum albumin), and combinations thereof. Useful pH ranges of said buffers may be from pH 4 to pH 10, more preferred from pH 5 to pH 9, most preferred from pH 6 to pH 8.

Enzymatically degradable spacer for use in methods as disclosed herein comprise, for example, polysaccharides, proteins, peptides, depsipeptides, polyesters, nucleic acids, and derivatives thereof. Proteins, peptides, and depsipeptides used as enzymatically degradable spacer can be functionalized via side chain functional groups of amino acids to attach multimerization component and antigen binding molecule. Side chains functional groups suitable for modification are for instance amino groups provided by lysine or thiol groups provided by cysteine after reduction of disulfide bridges.

Enzymatically degradable material for use in methods as disclosed herein comprise, for example, polysaccharides such as dextran, amylose, inulin, and cellulose, proteins, peptides, depsipeptides, polyesters, nucleic acids, and derivatives thereof, and wherein the enzyme used for degrading may be selected from the group consisting of glycosidases, dextranases, pullulanases, amylases, inulinases, cellulases, hemicellulases, pectinases, chitosanases, chitinases, proteinases, esterases, lipases, and nucleases.

### Embodiments

The method of the invention can be used for various applications in research, diagnostics and cellular therapy.

In one embodiment, the method can be used for the reversible labeling of cells in solution. Primary cells or cell lines are cultured in a suitable cell culture medium and a suitable container such as a plate, shaker flask, or a bioreactor. Cells comprising the target antigen are specifically labeled by addition of the antigen binding molecule that may be monomeric as well as multimerized. A detection moiety is coupled to the antigen binding molecule or the multimerization component. The cells are analyzed for the detection moiety by an appropriate method, e.g. FACS™ for a fluorescent label. Optionally, these cells can be isolated by an appropriate method, e.g. fluorescent sorting or magnetic isolation. Afterwards the label is removed by at least one washing step. In the case of a multimerized antigen binding molecule, the multimerization is disrupted before the washing step by an appropriate method as disclosed in the invention, for example by addition of a competing molecule that competes with multimerization component for the binding to the antigen binding molecule or that competes with the antigen binding molecule for the binding to multimerization component, or by chemical or enzymatic disruption within the multimerization component F, or by chemical or enzymatic disruption of the covalent bound of the multimerization component and the antigen binding molecule, or by combinations thereof. The kind of disruption depends on the covalent or non-covalent character of couplings. After removal of the label and the antigen binding molecule, the cells can be further cultured. Optionally, the cells can be again labeled reversibly with the same or with another specific antigen binding molecule. This procedure allows a multiple labeling with the option for a multiple sorting of cells. In one such example, a subsequent labeling of T cell epitopes is performed. The labeling of CD3 antigen in combination with either CD4 antigen or CD4 antigen together is difficult because the epitopes of the antigens are near to each other, resulting in possible epitope blocking by antigen binding molecules. Human peripheral blood mononuclear cells (PBMCs) are cultivated and labeled by an antigen binding molecule specific for CD3 that is coupled to a fluorochrome. Then the cells are analyzed, e.g. by flow cytometry. Optionally, CD3-positive cells can be isolated by fluorescent sorting. Afterwards, the antigen binding molecule and the label is removed from the CD3-positive cells by at least one washing step and optionally by disruption of the multimerized antigen binding molecule complex. Then antigen binding molecule(s) specific for CD4 and/or CD8, coupled to a fluorochrome, is/are added to the cells, and the cells are again analyzed and optionally sorted. Antigen binding molecule(s) specific for CD4 and/or CD8 may also be removed likewise. One advantage is that in a subsequent reversible labeling procedure, the same detection moiety can be used for different antigens. Another advantage is the generation of "label-free" cells that may be beneficial for cell therapeutic applications.

In another embodiment, the method can be used for the reversible labeling of fixed cells. The method is in principal similar to the reversible labeling of cells in solution, but cells or tissues are fixed by fixation agents such as formaldehyde, acetone, ethanol, or methanol, and optionally permeabilized by agents such as acetone or detergents, for analysis by microscopy and/or imaging. Optionally, said fixed cells may be embedded, for example in paraffin. Antigens of these fixed cells and also components of the extracellular matrix (for example extracellular matrix proteins) can be reversibly labeled. After removal of the antigen binding molecule and the label by at least one washing step, the next antigen binding molecule specific for the same or another antigen can be added for reversibly labeling. By this method, it is possible to serially label, detect and analyze hundreds of different antigens using a single sample without the need to use hundreds of different detection moieties and without the need to switch off detection moieties between serial additions of antigen binding molecule (e.g. by bleaching of fluorescent moieties).

In one embodiment, the reversible labeling of an antigen in a biological specimen is carried out by using a multimerization domain that has the capability to bind at least two monovalent antigen binding molecules. For some antigen binding molecules, the binding reaction can be enhanced due to the avidity effect of multimerized antigen binding molecules. In one example, the multimerization domain is an antibody directed against a polypeptide tag such as a poly-histidine tag, or a FLAG tag that is part of the antigen binding molecule, or against biotin, wherein the antigen binding molecule is biotinylated. In another example, the multimerization domain is avidin, streptavidin, or a mutant thereof, while the antigen binding molecule is biotinylated or carries a polypeptide that mimics biotin. In another example, the multimerization domain is a polymer such as a polysaccharide (for example dextran), wherein at least two antigen binding molecules are coupled to one multimerization domain. In some embodiments, the polymer can be degraded mechanically, chemically or by enzymatic means, and/or the coupling between the multimerization domain and the antigen binding molecules can be disrupted by addition of a competing agent and/or by mechanical, chemical or by enzymatic disruption of the coupling. One example comprises an antigen binding molecule that comprises a biotin-mimicking peptide, and free biotin as a competing agent, both competing for the binding to streptavidin or variants thereof (multimerization domain). A detection moiety such as a fluorochrome may be coupled to streptavidin or variants thereof. Another example for a competing agent comprises EDTA that competes with the multimerization domain and/or the antigen binding molecules that are coupled to each other in a metal ion-dependent manner. In one embodiment, the conformation of the multimerization domain, coupled to antigen binding molecules, is changed by a stimulus in order to monomerize antigen binding molecules. For example, calmodulin or variants thereof may be inserted in the antigen binding molecule, and adding or removing Ca²⁺ induces a conformational change of the multimerization component due to the conformational change of calmodulin or variants thereof within the antigen binding molecule. The conformational change of the multimerization component disrupts the binding of the monovalent antigen binding molecules to the multimerization component, and as a result the monovalent antigen binding molecules become monomerized.

In all embodiments, the detection moiety may be coupled to the multimerization domain and/or the antigen binding molecule.

In one embodiment, the disruption of the multimerization complex in order to monomerize the antigen binding molecules is conducted using at least one of the following exemplary methods: (i) Enzymatic or chemical disruption of the multimerization component, for example enzymatic digestion of dextran by the enzyme dextranase. (ii) Mechanical disruption by for example shear forces to interrupt the non-covalent binding between the multimerization component and the antigen binding molecule. (iii) Addition of a competing reagent to interrupt the non-covalent binding between the multimerization component and the antigen binding molecule, for example free biotin as competitor to the binding of biotinylated antigen binding molecule and an anti-biotin antibody, wherein said antibody may be bound to a further reagent or moiety such as dextran. (iv) Addition of a chelating reagent such as EDTA or EGTA to interrupt the non-covalent binding between the multimerization component and the antigen binding molecule, for example the binding between a poly-histidine sequence in the antigen binding molecule and a metal chelate such as Ni-NTA, or when the binding is Ca²⁺ dependent. (v) Changing a physical parameter such as pH value, temperature, and/or ionic strength to interrupt the non-covalent binding between the multimerization component and the antigen binding molecule, for example by using a pH-sensitive binding interaction that may be formed at pH values of 7 and higher and that may be disrupted at pH values of less than 7. (vi) Induction of a conformational change to interrupt the non-covalent binding between the multimerization component and the antigen binding molecule. The conformational change may be in the multimerization component, the antigen binding molecule, or both. In one example, the antigen binding molecule is a scFv, and the linker region connecting light and heavy chain of the scFv is calmodulin or a variant thereof. Calmodulin is known to change its conformation by the addition of Ca²⁺. This change in conformation allows calmodulin to bind many different target proteins. M13 is a peptide derived from a target protein that is bound by calmodulin in the presence of Ca²⁺. The antigen is contacted with the scFv, which is optionally bound to a detection moiety, in the absence of Ca²⁺ and M13 peptide (or a peptide of similar function). The complex may be analyzed and/or isolated. Addition of Ca²⁺ and/or M13 peptide induces the change in conformation, thereby disrupting the non-covalent binding between the multimerization component and the scFv. Alternatively, a small molecule may be used instead of M13 peptide. (vii) Enzymatic or chemical disruption to interrupt the covalent binding between the multimerization component and the antigen binding molecule, for example chemical or photoinduced cleavage of a linker, or reduction of a disulfide bond between multimerization component and the antigen binding molecule. These methods may be combined to reach an even higher disruption efficiency, for example up to 100%, meaning that all antigen binding molecules bind monovalently after disruption.

In one embodiment, the complex of antigen and monovalent antigen binding molecule is analyzed and/or isolated. Methods for analysis comprise, for example, flow cytometry, fluorescence microscopy, detection of phosphorescence, luminescence, magnetism, radioactivity, and light absorption. Isolation can be achieved by, for example, magnetic cell sorting (MACS®), and flow sorting (e.g. FACS™ or MEMS-based cell sorter), and microfluidic devices; for tissues, this may be combined with a tissue dissociation procedure and the isolation of cells by, for example, centrifugation to separate large cells from small cells and dense cells from light cells, the specific adherence of cells to glass, plastic, or to affinity matrices (for example a cell-specific antibody coupled to a matrix), and by laser capture microdissection. The detection moiety generally can be used for isolation of the complex according to their detection signal by optical means, electrostatic forces, piezoelectric forces, mechanical separation, acoustic means or magnetic forces. In addition, isolation of the complex can be conducted using solid-support moieties possessing a property or function which can be used for isolation purposes. The solid-support may be any of the known systems in biotechnology for immobilizing cells and can have the shape of particles, for example, sheets, plates, membranes, tubes, columns, wells, or micro arrays manufactured from various materials like polystyrene (PS), polymethylmethacrylate (PMMA), polyvinyl toluene (PVT), polyethylene (PE), or polypropylene (PP). Suitable materials are commercially available. The solid support can further be a nano- to microscale magnetic particle, also known in the art as magnetic bead. The mean diameter of the beads can range from 10 nm to 10 µm. Biocompatible magnetic particles are commercially available and consist of, for example, forms of magnetically iron oxide coated by a shell of dextran molecules or silica. The solid support may also be polymers containing magnetic materials. Suitable particles are commercial available from Miltenyi Biotec GmbH, Germany under the trade name "MicroBeads" and "MACSiBeads" possessing a hydrodynamic diameter of 50-100 nm or 3-4 µm, respectively. Magnetic particles are detected magnetically, e.g., by magnetic relaxometry, magnetic resonance imaging (MRI), magnetic force microscopy (MFM), superconducting quantum interference devices (SQUIDs), magnetometer.

In one embodiment, the antigen is analyzed and/or isolated after removing the monovalent antigen binding molecules.

In one embodiment, a biological specimen comprising a target antigen is reversibly labeled with a monovalent Fab-fluorochrome conjugate. For contacting, the biological specimen sample comprising the antigen is incubated with a Fab-fluorochrome conjugate such as, for example, Fab-FITC (Fab-fluorescein isothiocyanate) for 1-30 min at 4-10 °C. Useful concentrations for Fab-FITC conjugate for reversible labeling are in the range of approximately 0.1-10 µg/mL. One example for a buffer for the incubation and further analysis is PEB buffer (PBS, 2 mM EDTA, 0.5% BSA, pH 7.2-7.4). The incubation time is in the range of 1-30 min, preferably in the range of 5-15 min. Optionally, further reagents for detection can be added, for example propidium iodide to identify dead cells. A typical volume for analysis is about 100 µL for 1E+06 - 1E+07 cells. Then the sample is analyzed and/or isolated, for example by flow cytometry and/or flow sorting. In the next step, unbound Fab-FITC conjugate is removed. Therefore, at least one washing step with a suitable buffer such as PEB buffer is applied. A typical volume is 1 mL for 1E+06 - 1E+07 cells per washing step, i.e. a tenfold higher volume than for analysis. The temperature of the washing step is in the range of 4-37 °C, preferably in the range of 15-25 °C. For cells in solution, a washing step may comprise the addition of 1 mL buffer, a centrifugation step to pellet the cells, and the removal of the supernatant. For fixed cells, cells in tissue and extracellular matrices, a washing step may comprise the addition of a tenfold excess of buffer onto the labeled sample, a mixing and dispensing of the added buffer in or on the sample, and the removal of the buffer.

In one embodiment, the reversibility of staining is quantified. For example, the antigen in a biological specimen is contacted with a monovalent antigen binding molecule coupled with a detection moiety. The detectable moiety in the biological specimen is measured by an appropriate method such as flow cytometry for a fluorescent moiety or isotope counting for isotope mass tag moiety (measurement point #1). Afterwards, the monovalent antigen binding molecule coupled with a detection moiety is removed from the antigen within a defined time period. Said time period is preferably not longer than 4 hours, more preferred not longer than 2 hours, more preferred not longer than 1 hour, and most preferred not longer than 30 minutes. The remaining detectable moiety in the biological specimen is measured again by the same method (measurement point #2). The determined values of #1 and #2 are compared.

In one embodiment, the antigen binding molecule binds more than one antigen monovalently, for example bi-, tri- or multispecific antibodies. This may be useful when a biological specimen comprises more than one antigen that may be reversibly labeled. When the first antigen is contacted with a multispecific antigen binding molecule, this binding is monovalently. However, when the second antigen is bound by the same multispecific antigen binding molecule while the complex of first antigen and multispecific antigen binding molecule exists, avidity of binding is increased. This may be useful for the reversible labeling of biological specimen comprising more than one antigen using only one reagent instead of two separate reagents. Furthermore, by using a multispecific antigen binding molecule that bind with low affinity (for example, K_{D} ≥ 0.5E-08 M and k(off) ≥ 1E-03 sec-1) to each antigen, a specific labeling of a biological specimen occurs only when all antigens in the biological specimen are bound at the same time by the multispecific antigen binding molecule. This may be useful for the selective targeting and reversibly labeling of, for example, immune cells or tumor cells that comprise a specific combination of at least two antigens.

In one embodiment the detection moiety is coupled after the step of contacting a biological specimen comprising an antigen with a monovalent antigen binding molecule. In one example, the antigen is contacted with the monovalent antigen binding molecule (without detection moiety). When the complex of antigen and antigen binding molecule has formed, the detection moiety is added and coupled to the antigen binding molecule. A specific coupling of the detection moiety to the antigen binding molecule may be achieved by, for example, site-specific click chemistry or by using a detection moiety comprising a detection label that is coupled to a molecule that is able to bind specifically to the antigen binding molecule (such as a Fab molecule, coupled to a detection label, that is able to bind a specific epitope or amino acid sequence in the antigen binding molecule). In another example, the antigen is contacted with a complex comprising at least two monovalent antigen binding molecules and a multimerization component that leads to a multivalent (at least bivalent) binding of the antigen binding molecule to the antigen. When the complex of antigen, antigen binding molecules and multimerization component has formed, the detection moiety is added and coupled to the antigen binding molecule and/or multimerization component. Preferably, the detection moiety is coupled to the multimerization component. Again, this coupling may be achieved by site-specific coupling, for example by using a detection moiety comprising a detection label that is coupled to a molecule that is able to bind specifically to the multimerization component (such as an anti-dextran antibody, coupled to a detection label, in combination with a multimerization component comprising dextran). Coupling of the detection moiety after the step of contacting may be useful when the detection moiety is unstable under the conditions of contacting and therefore prolonged incubation periods of the biological specimen with the detection moiety should be minimized.

In one embodiment, the coupling of molecules may be a direct coupling. Examples for molecules that may be coupled are: (i) the detection moiety to the antigen binding molecule, (ii) the detection moiety to the multimerization component, (iii) the antigen binding molecule to the multimerization component. A direct coupling may be achieved by a covalent binding, for example, by a chemical bond, or a non-covalent binding, for example a ionic interaction, between the two molecules. Accordingly, the coupling of said molecules may be an indirect coupling. Said indirect coupling may be achieved by a covalent binding of a further reagent two both molecules, for example a linker, or by a non-covalent binding for example by using biotin coupled to one molecule and avidin coupled to the other molecule, wherein the indirect coupling is achieved by the binding of biotin and avidin. Another example for indirect coupling makes us of dextran as a multimerization component and an anti-dextran antibody coupled to a fluorophore. Direct and indirect couplings may be combined.

In one embodiment, the coupling of molecules may be a covalent coupling. Examples for molecules that may be coupled are: (i) the detection moiety to the antigen binding molecule, (ii) the detection moiety to the multimerization component, (iii) the antigen binding molecule to the multimerization component. A covalent coupling may be achieved by, for example, a chemical bond. Accordingly, the coupling of said molecules may be a non-covalent coupling. Said non-covalent coupling may be achieved by the binding of the molecules to each other by, for example, ionic and hydrophobic interactions. Covalent and non-covalent couplings may be combined.

In one embodiment, an antigen binding molecule is modified to change its kinetic values of antigen binding. More specifically, if an antigen binding molecule cannot be removed from its antigen after binding due to its kinetic values (k(on), k(off), K_{D}), the modification of said antigen binding molecule leads to a K_{D} value of equal or greater than 0.5E-08 M plus a k(off) value of equal or greater than 1E-03 sec-1, resulting in a modified variant that can be removed from its antigen. This can be achieved by changing the k(on) value, the k(off) value, or both values. Said modifications comprise substitutions, insertions, and/or deletions of at least one amino acid residue of the antigen binding molecule, and/or chemical and/or enzymatic modification of at least one amino acid residue, for example addition of a carbohydrate (e.g. glycosylation) or coupling of a chemical group (e.g. acylation, nitration). The "LAV" variants as described in the present invention are examples that were generated by substitution of one or more amino acid residues to modify the kinetic values in order to generate Fab variants that can be removed from the antigen after binding.

In one embodiment, the method of reversible labeling an antigen of a biological specimen comprising said antigen is combined with further downstream applications of the biological specimen. This is especially useful in applications where a remaining label results in undesirably results. One example is the culturing of labeled cells under conditions where the cells are in contact with the complement system. This can be the case in an *in vitro* cell culture system as well as when the cells are administered *in vivo.* Antibody or fragments thereof bound to cells may activate the complement pathway, resulting in death of these cells. The method of reversible labeling the antigen and removing the label (e.g. a Fab fragment coupled to a fluorochrome) before contacting the cell comprising said antigen with the complement system has the advantage that these cells are not attacked by the complement system.

In one embodiment, the monovalent antigen binding molecule may be part of a chimeric antigen receptor (CAR). Said CAR may comprise the antigen binding molecule, a transmembrane domain, and at least one endodomain, and it may be an isolated molecule or expressed on the surface of a cell. The antigen is preferably expressed by a tumor cell and located on the cell surface and/or inside the cell. The cell comprising the antigen is contacted with a CAR capable of binding the antigen. Using a detection moiety bound or coupled to the CAR, the tumor cell bound to the CAR may be reversibly labeled, analyzed and/or isolated. Thereby, the tumor cell may be reversibly labeled *in vitro* and *in vivo.* In one example, multispecific CARs with at least two monovalent antigen binding molecules may be used. This may be a bispecific CAR that binds monovalently to CD30 antigen and monovalently to a tumor antigen (such as carcino-embryonic antigen) in order to increase T cell activity, or a trispecific CAR that binds to three tumor antigens monovalently in order to prevent tumor antigen escape variants that otherwise result in relapses. For *in vitro* applications, the CAR may be removed from the antigen, for example to investigate cellular changes in a tumor cell after CAR removal.

### Examples

### Example 1: Generation of Fab fragments from monoclonal antibodies by papain digestion.

Fab fragments from monoclonal antibodies were generated using the Pierce Fab Preparation Kit (ThermoFisher). Therefore, purified monoclonal hybridoma antibodies (approx. 8 mg/mL) were treated with immobilized papain in cysteine-containing buffer pH 7 at 37 °C for 4 hours. Afterwards, undigested Ig antibody and the Fc part were removed by Protein A chromatography using a NAb Protein A Plus Spin Column. The Fab-containing flow-through fraction was further purified by size exclusion chromatography using a Superdex 16/60 column in PBS buffers at 0.5 mL/min flow-rate. Fab-containing fractions were identified by absorption measurement at 280 nm and pooled. Protein concentration of the papain-generated Fab was also determined at 280 nm. Purity was determined by SDS-PAGE (reducing conditions) followed by Coomassie staining (Table 1). FIG 2 exemplarily show a Coomassie-stained SDS-PAGE of the Fab generation from clone SJ25-C1.

**Table 1: Fab molecules produced by papain digestion of monoclonal antibodies ("pFab").**

| **Specificity** | **Clone** | **Fab variant** | **Fab purity** |
|---|---|---|---|
| CD4 | M-T466 | M-T466 pFab | 97% |
| CD8 | OKT8 | OKT8 pFab | 98% |
| CD14 | TÜK4 | TÜK4 pFab | 95% |
| CD19 | SJ25-C1 | SJ25-C1 pFab | 97% |
| CD25 | 4E3 | 4E3 pFab | 98% |
| CD25 | 3G10 | 3G10 pFab | 98% |
| CD27 | M-T271 | M-T271 pFab | 95% |
| CD45RA | T6D11 | T6D11 pFab | 97% |
| CD62L | 145/15 | 145/15 pFab | 97% |
| CD127 | MB15-18C9 | MB15-18C9 pFab | 98% |

### Example 2: Cloning of DNA vectors and transformation of E. coli.

For the expression of Fab fragments in *E.coli*, synthetic genes encoding for heavy chain and light chain variable domains (designated as VH and VL) of a humanized variant of anti-CD3 clone OKT3 (OKT3-hum), a mutated variant of anti-CD4 clone 13B8.2 (13B8.2-LAV), anti-CD8 clone OKT8, a mutated variant of anti-CD8 clone OKT8 (OKT8-LAV), anti-CD25 clone RFT5, a mutated variant of anti-CD25 clone RFT5 (RFT5-LAV), a humanized variant of anti-CD28 clone 15E8 (15E8-hum), a mutated variant of anti-CD45RA clone MEM56 (MEM56-LAV), for human kappa light chain constant region (designated as CL, SEQ ID NO:22), and for human IgG1 heavy chain constant region CH1 (SEQ ID NO:21) were designed using a standard software tool. For other SEQ ID NO, see FIG 12 (Table 3). Compatible flanking restriction sites were added to the synthetic genes sequences to facilitate direct cloning into an *E.coli* expression vector. Gene synthesis was performed externally by a service provider. The gene sequence encoding for the light chain (LC) variable domain (VL and CL) of the Fab fragment comprises a phoA signal sequence for periplasmatic translocation of the light chain. The gene sequence encoding for the heavy chain (HC) variable domain (VH and CH1, optionally followed by a poly-histidine tag) of the Fab fragment comprises a pelB signal sequence for periplasmatic translocation of the heavy chain. Cloning was performed using standard techniques known in the art. A resulting *E.coli* vector exemplarily shown for the expression of an anti-CD3 Fab is shown in FIG 3. All vector regions affected by the cloning procedure were validated by DNA sequencing.

### Example 3: Expression of recombinant Fab fragments in E.coli.

For production of recombinant Fabs specific for antigens described in example 2, *E.coli* W3110 cells were transformed with an appropriate expression vector such as, for example, "pOPE313 huCD3" which encodes for a humanized Fab variant against CD3. Pre-cultures of transformed *E.coli* were grown in complex media (containing soy-peptone, yeast extract and NaCl, supplemented with glucose) in shake flasks over night at 37°C. The next morning, a stirrable lab-scale bioreactor (3 L) containing complex media (containing soy-peptone, yeast extract and NaCl, supplemented with glucose as carbon source) was inoculated to a final optical density at 600 nm of 0.05 using the pre-culture. The cells were grown at 28°C and pH 7.0 with a pO₂ of at least 30%. Before induction the bioreactor was cooled to 25°C and target protein expression was subsequently induced by addition of 0.2 mM IPTG. The cultivation was continued for at least 20 h at 25°C. Glucose was constantly added as carbon source, and pH was kept at 7.0. Cell harvest was performed by centrifugation at 6000xg for 1 hour. Harvested cell pellets were stored at -20°C for further processing.

### Example 4: Purification of recombinant Fab fragments.

Recombinant Fabs were purified from fermented *E.coli* cells of example 3 by a periplasmic extraction step followed by chromatographic purification steps. For cell extraction, frozen *E.coli* pellets were resuspended in 10 mL of periplasmic extraction buffer (100 mM Tris, 10 mM EDTA) per 1 g cell pellet (wet weight) and incubated at 37°C with constant agitation for at least 16 hours. Afterwards the suspension was centrifuged at 6000xg for 1 hour. The supernatant contained the periplasmic fraction of *E.coli,* harboring the Fabs. Cell pellets were discarded. The supernatant was filtered and applied to a suitable chromatography column packed with an IMAC resin (for example ProCatchHis Resin, Miltenyi Biotec) for metal chelate affinity chromatography on an FPLC system (ÄKTA) for a first affinity capture purification step. Impurities were washed away using imidazole-containing buffer (5 mM). The target protein was eluted using a buffer containing an increased imidazole concentration of at least 50 mM. Eluted fractions were pooled and applied to a cation exchange matrix (for example SP sepharose). Elution was performed using a phosphate (25 mM) based buffer in the pH range of 6.0-7.4 containing NaCl in the range of 0.3-0.5 M. Eluates containing Fabs were pooled and the protein content was quantified by BCA assay and UV absorption (A₂₈₀ nm). Aliquots of purified Fabs were stored at -70°C towards use. In some cases, Fabs were rebuffered into, for example, PBS/EDTA. FIG 4 shows a Coomassie-stained SDS gel with samples of different steps of the purification process of Fab variant RFT5 rFab as an example, and FIG 5 shows the same for the Fab variant RFT5-LAV rFab. Fab purity, integrity (fully assembled Fab molecules) and the absence of aggregates was determined by SDS-PAGE under non-reducing conditions (Table 2). FIG 12 (Table 3) lists the used antibodies used their availability.

**Table 2: Fab molecules produced by recombinant expression ("rFab"). "LAV" designates mutated variants with modified kinetic constants, "hum" indicates humanized variants.**

| **Specificity** | **based on clone** | **Fab variant** | **Fab purity** |
|---|---|---|---|
| CD3 | OKT3 | OKT3-hum rFab | 97% |
| CD4 | 13B8.2 | 13B8.2-LAV rFab | 97% |
| CD8 | OKT8 | OKT8-LAV rFab | 98% |
| CD25 | RFT5 | RFT5 rFab | 98% |
| CD25 | RFT5 | RFT5-LAV rFab | 97% |
| CD28 | 15E8 | 15E8-hum rFab | 97% |
| CD45RA | MEM56 | MEM56-LAV rFab | 98% |

### Example 5: Intact mass determination of Fab fragments using mass spectrometry.

Identity of purified Fabs of example 4 was analyzed by intact mass determination using LC-MS (liquid chromatography-mass spectrometry) under non-reducing conditions. 7 µL of Fab bulk was applied onto a Zorbax 300SB-C8 column. Separation was achieved by a propanol/acetonitrile gradient in the presence of 0.1% formic acid at a flow-rate of 0.4 mL/min. Fab-containing peaks were detected at 280 nm and further analyzed by ESI (electrospray ionization) using a micrOTOF-Q II (Bruker). FIG 6 shows a result of the LC-MS analysis, exemplified by the anti-CD25 RFT5 rFab. The measured Fab masses were conform to the expected Fab masses.

### Example 6: Determination of kinetic constants of Fab variants using recombinant antigens.

The Fab variants of example 1 and 4 were analyzed by surface plasmon resonance spectroscopy (Biacore™). Therefore, the specific recombinant antigen (extracellular domain, expressed in mammalian cells) for a Fab was immobilized on a CM5 chip by standard amine coupling. The Fab variants were used as analytes in HBS-EP buffer (0.01 M HEPES pH 7.4, 0.15 M NaCl, 3 mM EDTA, 0.005% v/v Surfactant P20) in a concentration of up to 2 µM Fab. Evaluation was conducted with the Biacore™ X-100 Evaluation Software using an 1:1 binding mass transfer model. FIG 13 (Table 4) shows the determined kinetic constants: the association rate constant k(on) [M-1 s-1], the dissociation rate constant k(off) [s-1], and the equilibrium dissociation constant K_{D} [M]. FIG 7 exemplarily depicts Biacore™ sensorgrams of the anti-CD25 Fabs RFT5 rFab (FIG 7A) and RFT5-LAV rFab (FIG 7B) against immobilized recombinant CD25 antigen. In addition, published values of the anti-CD4 Fab variants wild-type, 13B8.2-Y92A, and 13B8.2-F100A are shown (taken from US7482000).
Typical error margins of surface plasmon resonance measurements are in the range of +/- a factor of 2 for each kinetic constant. This can be concluded from the investigations of Rich et al., "A global benchmark study using affinity-based biosensors", Anal Biochem. (2009) 386, 194-216, especially figure 9A. This means that, for example, a k(on) of 4E+05 M-1 s-1 covers a range from 2E+05 M-1 s-1 to 8E+05 M-1 s-1, a k(off) of 2E-03 s-1 covers a range from 1E-03 s-1 to 4E-03 s-1, and a K_{D} of 6E-08 M-1 covers a range from 3E-08 M-1 to 12E-08 M-1.

### Example 7: Generation of Fab biotin conjugates for reversible labeling of antigens.

The Fab variants of example 1 and 4 were chemically conjugated to biotin using a thiol-reactive biotinylation reagent. Therefore the Fab molecule was incubated in MES buffer, 10 mM DTT for one hour time at 20-22 °C to reduce the disulfide bridge. Afterwards, the reduced Fab was purified by gel filtration in PE buffer (PBS pH 7.2-7.4,2 mM EDTA). The biotinylation reagent, for example maleimide-PEO2-biotin (ThermoFisher Scientific), was dissolved in PEB buffer (PBS, 2 mM EDTA, 0.5% BSA, pH 7.2-7.4) at 5 mg/mL and added with a molar excess of, for example, 20-fold to the reduced Fab (2.5 mg Fab/mL). After incubation for 15 h at 20-22 °C, free (unreacted) biotin was removed by gel filtration utilizing PE buffer. Protein concentration was determined by the absorbance at 280 nm using the theoretically or experimentally determined extinction coefficient. The approximate biotin to protein ratio (B/P) was analyzed by a colorimetric determination using HABA (4'-hydroxyazobenzene-2-carboxylic acid, ThermoFisher Scientific).

### Example 8: Reversible labeling of antigens on cell surfaces.

Biotinylated Fab molecules of example 7 were used for reversible labeling of antigens (cell surface marker). The Fabs were contacted with the target cell comprising the specific antigen, and removed from the cells by one to five consecutive washing steps. SA-APC (streptavidin-allophycocyanin) was used as a detectable label for analysis at the step of contacting and after washing to remove the Fabs from the antigen, respectively. In this example, an indirect coupling of antigen binding molecule (Fab) and detection moiety (APC) was performed using biotin and streptavidin as third and fourth reagents. More specifically, 100 µL of human PBMCs (1E+07 cells) were incubated with 1 µg biotinylated Fab/mL for 10 min at 4-10 °C. The mixture was then divided and one subsample was washed five times with a tenfold volume of PEB buffer (PBS, 2 mM EDTA, 0.5% BSA, pH 7.2-7.4) at 20-25 °C: after addition of 1 mL PEB buffer, the cells were incubated for 2 min and then pelleted by centrifugation for 5 min, the supernatant was removed, and the cells were resuspended in 1 mL PEB buffer. This was repeated for a total of five washing steps. This processed subsample and the other subsample were analyzed: SA-APC (streptavidin-allophycocyanin) was added with a molar ratio of SA-APC to Fab of 4:1, and the mixture was incubated for 10 min at 4-10 °C. To remove unbound SA-APC, a ten- to twentyfold volume of PEB buffer, 4-10 °C, was added, the cells were immediately pelleted by centrifugation for 5 min, the supernatant was removed, and the cells were resuspended in 1 mL PEB buffer immediately before flow cytometric analysis, i.e. PBMCs without washing step (column "L" in FIG 8, i.e. labeling of antigen with Fab) and with five washing steps to remove Fab molecules (column "LR" in FIG 8) were analyzed (gated on living lymphocytes, granulocytes, and monocytes). The median of the SA-APC signal and the frequency of labeled cells was determined. PBMCs without biotinylated Fab was likewise incubated with SA-APC and served as a control, i.e. the background signal (column "C" in FIG 8, column "median of control (background)" in FIG 14 (Table 5)). FIG 8 shows exemplarily the dot plots for Fab molecules against, CD4 (M-T466), CD8 (OKT8, OKT8-LAV), CD25 (RFT5, RFT5-LAV), CD27 (M-T271), CD28 (15E8-hum), and CD45RA (T6D11). FIG 8A comprises examples for Fabs that enable reversible labeling, while the Fabs in FIG 8B do not enable reversible labeling. The antigens on human PBMCs were successfully labeled with Fab and APC that was coupled indirectly to the Fab after binding of the Fab to the antigen. In this way, there was no multimerization of the Fab before the binding of the antigen. In 11 out of 17 cases, the Fab was removed from the antigen by washing (i.e. these Fabs enable reversible labeling). It can be seen that for Fabs that enable reversible labeling, the remaining signal after washing to remove Fab ("LR") is a) very similar to the background signal ("C"), and b) completely differs from the signal at the step of contacting the cell comprising the antigen with the Fab ("L"). In contrast, for Fabs that do not enable reversible labeling, the remaining signal after washing to remove Fab ("LR") completely differs from the background signal ("C").

Furthermore, the effect of different numbers of washing steps for removal of Fab molecules was investigated. Therefore, the antigens were labeled and analyzed by flow cytometry as before, but here the PBMCs were washed one time and five times with PEB buffer at 20-25 °C, respectively. Again, the antigens were analyzed by flow cytometry (column "L": without washing step; column "LR1": one washing step; column "LR5": five washing steps, FIG 9). FIG 9 shows exemplarily the dot plots for Fab molecules against CD25 (RFT5-LAV) and CD28 (15E8-hum).

FIG 14 (Table 5) compiles the results of the flow cytometric analyses. For Fab molecules that enable reversible labeling, the reduction of the detectable label after washing to remove Fabs was at least 89%, i.e. the remaining signal (ii) was equal or less than 11% of the signal of the labeled antigen (i). For 10 out of 11 Fabs, the signal reduction was more than 90% after Fab removal, for 8 Fabs it was more than 95%, and for 4 Fabs it was more than 99%. For Fabs that do not enable reversible labeling, the reduction of the detectable label after washing to remove Fabs was in the range of 0% - 50%. An exception was T6D11 that reached 84% reduction; however, the remaining detectable label after washing is more than 100-fold over the background signal (median of 17.4 vs 0.1, i.e. still a specific signal of Fab bound to its target antigen), therefore T6D11 cannot be completely removed from the antigen and thus is classified as "not enabling reversible labeling". For comparison, the remaining detectable label after removal of Fabs that enable reversible labeling showed a maximum value of 1.2 with a background signal of 0.2 (SJ25-C1, 15E8-hum). For 13B8.2 wild-type Fab and Fab variants 13B8.2-Y92A and 13B8.2-F100A, results regarding substantial residual cell surface bound Fab/reversibility of labeling were taken from US9023604; there it is disclosed that a Fab is suitable for reversible labeling if no substantial residual Fab bound to the cell surface can be observed (no numbers or values such as median values were disclosed to define Fabs that enable reversible labeling).

In Table 6, the effect of different numbers of washing steps on the reduction of the detectable label is listed for two Fabs. It was shown that one washing step was sufficient to achieve a reversible labeling. By increasing the number of washing steps from one (iia) to five (iib), the efficiency of Fab and label removal was only slightly increased: For 15E8-hum, the signal decreased from 1.29 to 1.20 (i.e. from 4.3% to 4.0%, compared to the labeling signal), and for RFT5-LAV from 0.17 to 0.14 (from 13.8% to 11.4%).

**Table 6: Median values of detected label during labeling of antigens on the cell surface by Fabs (i) and after removal of the Fabs using one (iia) or five (iib) washing steps, respectively.**

| **Fab** | **median value (i)** | **median value after Fab removal, one washing step (iia)** | **median value after Fab removal, five washing steps (iib)** |
|---|---|---|---|
| 15E8-hum rFab | 30 | 1.29 | 1.20 |
| RFT5-LAV rFab | 1.23 | 0.17 | 0.14 |

### Example 9: Correlation of kinetic constants and reversible antigen labeling on biological specimen.

The kinetic parameters determined in example 6 and the flow cytometric data regarding reversible labeling of target antigens of example 8 were analyzed for correlations. Therefore, the kinetic data of analyzed Fab fragments were plotted as K_{D} vs k(on), K_{D} vs k(off) and k(off) vs k(on), and it was marked whether a Fab fragment is usable for reversible labeling of antigens or not. It was evident that reversible labeling is independent of the k(on) value. Surprisingly, all Fabs that enable reversible labeling were found within an area limited by a lower K_{D} and a lower k(off) value, which becomes evident when plotting K_{D} vs k(off) (Figure 10A). Fabs that do not enable reversible labeling were found outside of this area (Figure 10B).

Taken together, it was found that for reversible labeling of target antigens in biological specimen, the kinetic characteristics of an antigen binding molecule to the antigen are defined by an area limited by
a) K_{D} of equal or greater than 0.5E-08 M, and
b) k(off) of equal or greater than 1E-03 sec-1.

Upper limits for these kinetic parameters are in the magnitude of about 1E-04 M for K_{D} and about 1E-00 sec-1 for k(off).

### Example 10: Reversible labeling of antigens using a multimeric Fab complex.

Biotinylated Fab molecules of example 7 were used for reversible labeling of antigens (cell surface marker) in combination with a multimerization component to generate a complex of Fab, multimerization component, and detection moiety for a reversible labeling. In this example, biotinylated M-T466 pFab specific for CD4 antigen was used in combination with an anti-biotin antibody that served as multimerization component, wherein the antibody was directly coupled to PE (phycoerythrin) as detection moiety (anti-Biotin-PE, Miltenyi Biotec). Therefore, 2 µg biotinylated Fab/mL was incubated with anti-Biotin-PE in a molar ratio of 1:2 for 10 min at 4-10 °C to generate to complex of Fabs, multimerization domain and detection moiety. Afterwards human PBMCs (1E+07 cells) were added and the mixture was incubated for 10 min at 4-10 °C. Then the cells were washed with PEB buffer (PBS, 2 mM EDTA, 0.5% BSA, pH 7.2-7.4) at 0-4 °C to remove unbound Fab / anti-Biotin-PE complexes and unbound anti-Biotin-PE molecules: after addition of 1 mL PEB buffer (0-4 °C), the cells were immediately pelleted by centrifugation for 5 min, the supernatant was removed, and the cells were resuspended in 1 mL PEB buffer (0-4 °C). The sample was divided, and one subsample was further incubated with 2 mM biotin for 10 min at 20-25 °C to disrupt the multimerized complex and to monomerize Fab molecules in order to remove these from the antigen. By further pelleting the cells by centrifugation for 5 min at 20-25 °C, removing the supernatant to remove monomerized Fab, and resuspending the cells in 1 mL PEB buffer, this also serves as a washing step. Both subsamples were analyzed by flow cytometry for the PE label (gated on living lymphocytes). The median of the PE signal and the frequency of labeled cells was determined. PBMCs without biotinylated Fab was likewise incubated with anti-Biotin-PE and served as a control, i.e. the background signal. FIG 11 shows the dot plots for the Fab molecule specific for CD4 (M-T466): "L", labeling of antigen with multimerized Fab; "LR", after disruption of the multimerized complex with free biotin and a washing step to remove monomerized and dissociated Fab; "C": control (background signal). The CD4 antigen on human PBMCs was successfully labeled with the complex of multimerized biotinylated Fab and anti-Biotin-PE (median value of 16.4), and Fab and PE label were successfully removed after disruption of the complex, resulting in a median value of 0.2 that is similar to the background.

## Claims

1. A method for reversible labeling an antigen A of a biological specimen comprising said antigen A with a monovalent antigen binding molecule K comprising a binding site B for specifically binding said antigen A, the method comprising the steps
a) contacting said biological specimen comprising said antigen A with said antigen binding molecule K, thereby generating a complex of A/K,
b) removing the monovalent antigen binding molecule K that is not bound in said complex of A/K, wherein the equilibrium dissociation constant (K_{D}) for the binding of said antigen A and said monovalent antigen binding molecule K has a value of equal or greater than 0.5E-08 M and wherein the dissociation rate constant (k(off)) of said binding has a value of equal or greater than 1E-03 sec-1.

2. The method according to claim 1, wherein the value for K_{D} is in the range of 0.5E-08 M and 1E-04 M, and wherein the value for k(off) is in the range of 1E-03 sec-1 and 1E-00 sec-1.

3. The method according to claim 1 or 2, wherein said antigen binding molecule K is coupled to a detection moiety D, thereby allowing to analyze and/or isolate said complex of A/K between step a) and b) and/or after step b).

4. The method according to claim 3, wherein said detection moiety D is selected from the group consisting of chromophore, fluorescent moiety, phosphorescent moiety, luminescent moiety, light absorbing moiety, radioactive moiety, transition metal isotope mass tag moiety, and magnetic particle.

5. The method according to any one of claims 1 to 4, wherein said removing is performed by at least one washing step.

6. A method for reversible labeling an antigen A of a biological specimen comprising said antigen A with a monovalent antigen binding molecule K comprising a binding site B for specifically binding said antigen A and at least one binding site Y for coupling to a multimerization component F comprising at least two binding sites G for coupling to said monovalent antigen binding molecule K, wherein said multimerization component F is coupled to a detection moiety D, wherein the assembly of the complex of F/K comprises at least two of said antigen binding molecule K coupled to said multimerization component F, and wherein F and K are coupled non-covalently via the binding sites G and Y,
the method comprising the steps
a) contacting
i) said biological specimen comprising said antigen A with said monovalent antigen binding molecule K, thereby generating a complex of A/K, and subsequently contacting said complex of A/K with said multimerization component F, thereby generating a complex of A/K/F; or
ii) said biological specimen comprising said antigen A with said complex of F/K, wherein said assembly of F/K is performed before step a), thereby generating a complex of A/K/F,
b) analyzing and/or isolating said complex of A/K/F,
c) disrupting the complex of F/K, thereby monomerizing said at least two of said antigen binding molecule K,
d) removing the monovalent antigen binding molecule K that is not bound in said complex of A/K,
wherein the equilibrium dissociation constant (K_{D}) for the binding of said antigen A and said monovalent antigen binding molecule K comprising a binding site B has a value of equal or greater than 0.5E-08 M and wherein the dissociation rate constant (k(off)) of said binding has a value of equal or greater than 1E-03 sec-1.

7. The method according to claim 6, wherein the value for K_{D} is in the range of 0.5E-08 M and 1E-04 M, and wherein the value for k(off) is in the range of 1E-03 sec-1 and 1E-00 sec-1.

8. The method according to claim 6 or 7, wherein said detection moiety D is selected from the group consisting of chromophore, fluorescent moiety, phosphorescent moiety, luminescent moiety, light absorbing moiety, radioactive moiety, transition metal isotope mass tag moiety, and magnetic particle.

9. The method according to any one of claims 6 to 8, wherein said removing is performed by at least one washing step.

10. The method according to any one of claims 6 to 9, wherein said disruption is performed by
(i) contacting said complex of F/K in step c) with a molecule that competes with F for the binding to K or that competes with K for the binding to F, thereby replacing F or K in the complex of F/K, respectively; or
(ii) chemical or enzymatic disruption within the multimerization component F; or
(iii) a combination of (i) and (ii).

11. A method for reversible labeling an antigen A of a biological specimen comprising said antigen A with a monovalent antigen binding molecule K comprising a binding site B for specifically binding said antigen A, wherein at least two of said monovalent antigen binding molecule K are coupled to a multimerization component F, thereby generating a complex of F/K, wherein F and K are coupled covalently, wherein said multimerization component F is coupled to a detection moiety D, the method comprising the steps
a) contacting said biological specimen comprising said antigen A with said complex of F/K, thereby generating a complex of A/K/F,
b) analyzing and/or isolating said complex of A/K/F,
c) disrupting the complex of F/K, thereby monomerizing said at least two of said antigen binding molecule K,
d) removing the monovalent antigen binding molecule K that is not bound in said complex of A/K,
wherein the equilibrium dissociation constant (K_{D}) for the binding of said antigen A and said monovalent antigen binding molecule K comprising a binding site B has a value of equal or greater than 0.5E-08 M and wherein the dissociation rate constant (k(off)) of said binding has a value of equal or greater than 1E-03 sec-1.

12. The method according to claim 11, wherein the value for K_{D} is in the range of 0.5E-08 M and 1E-04 M, and wherein the value for k(off) is in the range of 1E-03 sec-1 and 1E-00 sec-1.

13. The method according to claim 11 or 12, wherein said detection moiety D is selected from the group consisting of chromophore, fluorescent moiety, phosphorescent moiety, luminescent moiety, light absorbing moiety, radioactive moiety, transition metal isotope mass tag moiety, and magnetic particle.

14. The method according to any one of claims 11 to 13, wherein said removing is performed by at least one washing step.

15. The method according to any one of claims 11 to 14, wherein said disruption is performed by
(i) chemical or enzymatic disruption within the multimerization component F; or
(ii) chemical or enzymatic disruption of the covalent bound of K/F; or
(iii) a combination of (i) and (ii).
